(19) Europäisches Patentamt
European Patent Office
Office européen des brevets

(11) EP 2 921 159 B1

# (12) EUROPEAN PATENT SPECIFICATION

(45) Date of publication and mention of the grant of the patent:
**24.07.2019 Bulletin 2019/30**

(21) Application number: **14001048.9**

(22) Date of filing: **21.03.2014**

(51) Int Cl.:
*A61K 8/31* (2006.01) *A61K 8/37* (2006.01)
*A61K 8/39* (2006.01) *A61K 8/49* (2006.01)
*A61Q 1/14* (2006.01) *A61K 8/891* (2006.01)
*A61K 8/02* (2006.01) *A61K 8/06* (2006.01)

## (54) Cosmetic compositions as well as use of a cosmetic composition and method using same for removing waterproof make-up

Kosmetische Zusammensetzungen sowie Verwendung einer kosmetischen Zusammensetzung und Verfahren damit zum Entfernen von wasserfestem Make-up

Compositions cosmétiques ainsi que l’utilisation d’une composition cosmétique et son procédé d’utilisation pour l’élimination de maquillage étanche à l’eau

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(43) Date of publication of application:
**23.09.2015 Bulletin 2015/39**

(73) Proprietor: **Essity Operations France**
**93400 Saint-Ouen (FR)**

(72) Inventors:
- **Clermont, Anne-Gaëlle**
  **68320 Kunheim (FR)**
- **Hau, Caroline**
  **68320 Kunheim (FR)**
- **Bret, Bruno**
  **68320 Kunheim (FR)**

(74) Representative: **Essity Hygiene and Health AB et al**
**Essity IP Department**
**405 03 Göteborg (SE)**

(56) References cited:
**EP-A1- 1 602 359** **EP-A1- 2 505 180**
**EP-A2- 1 340 491** **US-A1- 2004 028 633**
**US-A1- 2004 081 679** **US-B1- 6 242 396**
**US-B1- 6 342 469**

- **Anonymous: "Polyglycerol Esters for successful cosmetic formulations", Hydrior in-cosmetics, 31 December 2006 (2006-12-31), XP002729774, Retrieved from the Internet: URL:http://www.in-cosmetics.com/__novadocuments/37619?v=635260873579430000 [retrieved on 2014-09-16]**

EP 2 921 159 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The present invention relates to a use of a cosmetic composition for removing, in particular waterproof, make-up, and to a method for removing such make-up by applying said cosmetic composition to the skin. Moreover, the present invention is concerned with a cosmetic composition, especially for removing make-up, in particular waterproof make-up, and to an applicator for cosmetic purpose impregnated or coated with said cosmetic composition.

BACKGROUND ART

While numerous compositions for removing make-up have been tested and many commercialized, the products for removing waterproof make-up, which is becoming more and more popular nowadays, leave significant room for improvement. Looking at products on the European market destined for the removal of waterproof make-up, the following types can be distinguished.

Firstly, there are oil-based products, for instance cleansing oils that may be offered as liquids in bottles or impregnated into non-wovens. An example of the latter non-woven-based products are the "Quickies" (trade name) marketed by Sara Lee H&BC France SNC. Quickies is a product having, coated onto or impregnated into a non-woven tissue, a composition comprising mineral oil (Paraffinum Liquidum), a hydrophilic emulsifier and butylated hydroxytoluene (BHT). While the Quickies product is quite effective in removing waterproof make-up, it leaves a greasy film on the face and on the fingers after its use and has a heavy and oily texture on the skin. Differently stated, customers felt an unpleasant sensation on the skin after using Quickies for removing make-up. To remove the greasy film, a subsequent rinsing with an aqueous solution is normally needed. The above factors are oftentimes regarded as unattractive by customers.

A second type of products currently on the European market for removing make-up, including waterproof make-up, are those based on a classical oil-in-water (O/W) emulsion. Such products are marketed for instance by SCA under the "Demak Up" (trade name) series. To give an example, while it is perceived by customers as not greasy and as having a pleasant fragrance, the "Demak Up Sensitive" (trade name) product is generally not effective enough for waterproof make-up.

Compositions for removing make-up are also described in the patent literature, as follows.

EP 1 618 925 A1 is concerned with cosmetic compositions for the removal of make-up, and water-resistant make-up is expressly mentioned. The cosmetic composition can be comprised in an applicator, for instance in the form of pads or wipes. According to Claim 1 of the reference, the cosmetic composition comprises among others at least one emulsion. According to Claim 5 and paragraph [0022] and [0045], the emulsion preferably is an O/W emulsion prepared by the Phase Inversion Technique (PIT). Such O/W emulsions are also referred to as PIT emulsions in the patent application. The distinct ingredients of the emulsion are generally described in connection with PIT emulsions. Water-in-oil (W/O) emulsions are only mentioned in passing in paragraph [0041] of the document.

US 6,342,469 B1 relates to a make-up removing and/or cleansing cosmetic composition and does not address the difficulties in removing waterproof make-up. The cosmetic compositions of the U.S. patent are directly applied to the skin for instance in the form of milks and may have a viscosity from 0.2 to 3 Pa·s.

The disclosure of US 6,344,204 B1 is similar to the above US 6,342,469 B1, but the purpose is more on the general cosmetic and/or dermatological application of the composition with make-up removal only mentioned in passing.

WO 01/56534 A1 is concerned with crystal clear, *i.e.* transparent, personal care and cosmetic products, without using substantial quantities of irritating substances. The cosmetic products can for instance be bath preparations, hair conditioners, cleansers and make-up removers. Applicators such as pads are not mentioned in the document.

Pursuant to Claim 1, EP 1 602 359 A1 relates to a cosmetic or dermatological cloth consisting of a single-ply non-woven wetted with a cosmetic and/or dermatological impregnation solution having a viscosity of less than 2,000 mPa·s. The cloth can be used for make-up removal. The impregnating solution may contain surfactants having an HLB of more than 25, preferably of more than 35. In particular, when used for removing make-up, the cloth is preferably free of such surfactants.

GB 2 000 969 A aims at obtaining stable W/O emulsions avoiding the phenomenon of conversion into an O/W emulsion. These emulsions are stated as being useful for producing cosmetic products which can be used for washing or skin care, and make-up removal milks, body milks and sun milks are given as examples.

The object of US 2003/0027738 A1 is the provision of an antibacterial preserving system for substrate-containing articles such as wipes, which is not irritant to the skin or the eyes. Pursuant to Claim 1, the U.S. patent application is concerned with a wipe comprising a non-woven substrate and a physiologically acceptable composition in contact with the substrate. The possible use of this wipe for cleansing and/or removing make-up is mentioned. O/W emulsions are preferred with numerous different presentation forms being mentioned in passing, including W/O emulsions (see paragraph [0026]).

In the following, some patent documents mentioning W/O emulsions for applications other than make-up removal are presented.

US 2005/0002994 A1 is concerned with a cosmetic or dermatological tissue comprising a water-insoluble non-woven which is impregnated and/or moistened with the cosmetic or dermatological W/O emulsion (Claim 1). The patent application talks about the potential use of the emulsions as cosmetic and dermatological preparations and as cleansing agents but does not mention make-up removal.

EP 1 555 017 A1 relates to a cosmetic or dermatological composition on the basis of a low-viscosity W/O emulsion (Claim 1), which composition is sprayable and nonetheless stable. Make-up removal is not an issue in the patent application, nor are applicators.

Another document describing W/O emulsions is EP 1 147 760 A2.

The emulsions in EP 1 340 491 A2 are high internal phase emulsions with an oil content which is at most 20% by weight and an aqueous phase which is at least 75 % by weight.

US2004/028633 A1 is directed to compositions for skin care and similar applications and comprises silicone-based oil. The oil content in the compositions disclosed in the Examples in US2004/028633 A1 is in the order of 20% by weight or less, with a water content in the order of 70-80% by weight.

EP 2 505 180 A1 is concerned with the preparation of microemulsions having low viscosity defined as a viscosity of ≤4000 mPas, preferably ≤2500 mPas and most preferred ≤500mPas. Examples of microemulsions in EP 2 505 180 A1 contain from 5.4 to 13 % of isopropylpalmitate and from 7.0 to 12.0 % of dicaprylylether the total amount of isopropylpalmitate and dicaprylylether not exceeding 20 % in any of the examples.

The compositions in DE10154627A1 are low viscosity compositions which are broadly defined as having a viscosity of less than 2,000 mPa·s. The compositions are used in skincare, cleansing or deodorant tissues.

US6,426,079B1 is directed to skin care and make-up compositions which are intended to be applied to skin and to stay on the skin and not smear onto other surfaces. The composition in US6,426,079B1 may contain 33.5% of an oil component being a mixture of cyclic silicone (cyclomethicone), isododecane and polydimethylsiloxane (dimethicone)with isododecane amounting to 13% of the oil component.

Against the background of the above situation on the market and the prior art, the invention aims at achieving the following effects in combination:

1) Excellent efficacy for removing, in particular waterproof, make-up;
2) No greasy film, heavy texture or unpleasant sensation on the skin after application of the product;
3) No need to rinse off, e.g. using an aqueous solution; and
4) Facile and homogeneous impregnation of applicators, such as pads and wipes.

## SUMMARY OF THE INVENTION

The above object is solved by a use of a cosmetic composition for removing make-up, in particular waterproof make-up, as recited in the appended Claim 1, as well as to a corresponding method for removing make-up as defined in Claim 10. A specific cosmetic composition excellently suitable for removing make-up, in particular waterproof make-up, is subject of the appended Claim 11, and an applicator for cosmetic purpose, such as a wipe or pad, impregnated or coated with said specific cosmetic composition is subject of Claim 13.

Preferred embodiments of the invention are subject of the dependent claims.

In the method for removing make-up according to Claim 10, the preferred embodiments of the applicator for cosmetic purpose and the components of the cosmetic composition are as defined in the dependent Claims 2 - 9. Also the preferred embodiments of the emulsifier component as in Claim 11 and the applicator for cosmetic purpose as in Claim 13 are according to preferred embodiments as defined in the dependent Use Claims 2 - 9.

## DETAILED DESCRIPTION OF THE INVENTION

The word "comprises" and "comprising" as used herein is to be construed in the sense of "contains" and "containing", respectively, and encompasses the more limiting expressions "essentially consists of"/"essentially consisting of", and "consists of"/"consisting of".

The present invention resides in the use of a cosmetic composition for removing make-up, in particular waterproof make-up, and a corresponding method for removing said make-up. As meant herein, make-up is waterproof when it cannot be removed with water and therefore has a long-lasting effect. Waterproof refers to the ability of the make-up to repel water and to display permanence with respect to water. The term "make-up" as used herein is construed broadly and covers for instance lipstick, eye liner, eye shadow, mascara and foundation. Accordingly, the waterproof make-up that is removed with particular benefit in the present invention, may be waterproof lipstick, waterproof eye liner, waterproof

eye shadow, waterproof mascara and waterproof foundation, and is preferably waterproof mascara or waterproof eye shadow, most preferably waterproof mascara.

In the present invention, the cosmetic composition is in the form of a water-in-oil (W/O) emulsion. Differently stated, the cosmetic composition consists of the W/O emulsion. In the W/O emulsion, an aqueous, *i.e.* water-based, phase is dispersed in an oil phase.

The W/O emulsion constituting the cosmetic composition for use in the present invention comprises three essential components, which will be described in more detail below.

1. Oil component

The first essential component is the oil component (i). The term "oil" is used herein for water-insoluble, organic, natural and synthetic, cosmetically useful oils, preferably having a liquid (also viscous) consistency at room temperature (23°C).

In the present invention, the oil component has a polarity of at least 20 mN/m. The polarity of oil can be determined using a ringtensiometer (*e.g.* Krüss K 10), measuring the boundary layer energy, which is the boundary layer tension in mN/m. The lower limit is 5 mN/m. The method is suitable for low viscosity liquids given that a boundary layer is present (that is the liquids are not miscible). The polarity of the oil is determined against water. One method for determining the boundary layer tension is the one described in the ASTM method D971-99a (reapproved 2004).

The following table lists the polarity for common oils.

| **Common Oils (CFTA-names)** | **Polarity index (mN/m)** |
|---|---|
| Non-polar | |
| Isoparaffin ($C_{12}$-$C_{14}$) | 53.0 |
| Squalan | 46.2 |
| Isohexadekan (ARLAMOL ND) | 43.8 |
| Mineral oil (Paraffin oil perliquidum | 43.7 |
| Mineral oil (Paraffin oil subliquidum | 38.3 |
| Polar | |
| Cetylstearyloctanoat (purceline oil) | 28.6 |
| Dimethicone (silicon oil 20 cSt) | 26.6 |
| Isopropylpalmitate | 25.2 |
| Octyldodecanol | 24.8 |
| Dioctyladipat (ARLAMOL DOA) | 24.5 |
| Isopropylmyristat | 24.2 |
| Octylpalmitate (2-ethylhexylpalmitate) | 23.1 |
| Hexamethyldisiloxan | 22.7 |
| Isopropylstearate | 21.9 |
| Capryl/caprine acid triglyceride (neutral oil) | 21.3 |
| Isopropylisostearate | 21.2 |
| Jojoba oil | 20.8 |
| Cyclomethicone (ARLAMOL D4) | 20.6 |
| Peanut oil | 20.5 |
| Almond oil | 20.3 |
| Sunflower oil | 19.3 |
| Decyloleate | 18.7 |
| Avocado oil | 18.3 |

(continued)

| Polar | |
|---|---|
| Olive oil | 16.9 |
| Castor oil | 13.7 |
| Calendula oil | 11.1 |
| Wheat germ oil | 8.3 |

The content of the oil component in the cosmetic composition of or for use in the present invention, relative to the total mass of the cosmetic composition, is 22 - 40 % by mass, and most preferably 25 - 35 % by mass.

The oil component comprises non-polar oil and low polarity oil. In line with the common understanding, oils with a polarity greater than 30 mN/m are considered as non-polar oils herein. Low polarity oils are defined in this specification as oils having a polarity in the range of from 20 to 30 mN/m.

The mass ratio non-polar oil/low polarity oil is in the range of from 1 to 5, more preferably in the range of from 2 to 3. According to a particularly preferred embodiment of the invention, the non-polar oil is a $C_{12}$ - $C_{16}$ alkane and the low polarity oil is a silicone oil or a dialkyl carbonate, most preferably a cyclic silicone, and the mass ratio of non-polar oil with respect to low polarity oil is in the range of from 2 to 3.

The non-polar oil of the oil component preferably is a hydrocarbon-based oil. In particular, the non-polar oil is a hydrocarbon having 8 to 32, preferably 12 to 25, more preferably 15 to 20 carbon atoms. The hydrocarbon that can be used as the non-polar oil in the present invention can be linear or branched. Further, the hydrocarbon can be a saturated hydrocarbon, *i.e.* an alkane, or an unsaturated hydrocarbon containing one or more unsaturations, in particular C-C-double bonds. Examples of unsaturated hydrocarbons are optionally hydrogenated polydecene and squalene.

Concretely, the following non-polar hydrocarbon-based oils can be used with preference in the present invention:

| **INCI name** | CAS **No.** | **Trade Name** | **Provider** |
|---|---|---|---|
| | | | |
| Paraffinum Liquidum | 042-47-5 | Huile de Paraffine (Perliquidum) | Centonze |
| Isohexadecane | 4390-04-9 | Arlamol HD | Croda |
| Isododecane | 93685-81-5 + 13475-82-6 | Isododecane | Ineos |
| Hydrogenated Polydecene | 68037-01-4 | Nexbase 2004 FG | Jan Dekker |

In a preferred embodiment, the non-polar oil is a linear or branched alkane having 8 to 32, preferably 12 to 25, more preferably 15 to 20 carbon atoms. Examples are squalane, paraffinic oils, isododecane, isohexadecane, isoeicosane, or dialkycyclohexane.

According to the most preferred embodiment, the non-polar oil is a $C_{12}$ to $C_{16}$ alkane, such as isododecane or isohexadecane.

Mineral oil, which usually refers to less viscous mixtures of hydrocarbons having from 16 to 20 carbon atoms, can also be used as the non-polar oil. For example, paraffinum liquidum can be used as the mineral oil.

The low polarity oil as meant herein can be a silicone oil or an ester. Without limitation, the ester can be selected from the following groups:

1) Dialkyl carbonates or dialkenyl carbonates;
2) Liquid synthetic glycerides;
3) Liquid linear or branched carboxylic acid esters.

1) The dialk(en)yl carbonates preferably have at least one C6 to 22 alkyl or alkenyl group (preferred total number of C atoms: not more than 45 including the C atom for the carbonate unit). The alkyl or alkenyl group can be straight or branched. The alkenyl unit may display more than one double bond. These carbonates can be obtained by transesterification of dimethyl or diethyl carbonate in the presence of C6 to C22 fatty alcohols according to known methods (cf. Chem. Rev. 96, 951 (1996)). Typical examples for dialk(en)ylcarbonates are the (partial) transesterification products of caprone alcohol, capryl alcohol, 2-ethylhexanol, n-decanol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linolyl alcohol, linolenyl alcohol, elaeostearyl alcohol, arachidyl

alcohol, gadoleyl alcohol, behenyl alcohol, erucyl alcohol and brassidyl alcohol as well as their technical mixture, which are for instance obtained by high pressure hydrogenisation of technical methyl esters on fat or oil basis. Particularly suitable for use in the invention are dihexyl-, dioctyl-, di-(2-ethylhexyl)- or dioleylcarbonate. Thus it is preferred to use either short chain (C6 to C10) alkyl or alkenyl carbonates.
According to the most preferred embodiment, the ester constituting the low polarity oil is dioctyl carbonate, also named dicaprylyl carbonate, which compound is available from BASF under the trade name Cetiol CC (CAS no. 1680-31-5).

2) The liquid synthetic glycerides are mono-, di- and/or tri esters (carboxylic acid esters) of glycerol (in particular di- and/or triesters). Preferably the carboxylic acid component has from 6 to 24, more preferably 6 to 18, in particular 8 to 18 carbon atoms. The carboxylic acid can be branched or unbranched as well as saturated or unsaturated, with saturated carboxylic acids being preferred.

3) The liquid linear or branched carboxylic acid esters are preferably fatty acid esters and more preferably esters of monovalent carboxylic acids having at least one long chain alkyl or acyl residue (each having at least 6 C atoms, in particular at least 12 C atoms). The carboxylic ester type emollient oils include those having more than 6 carbon atoms in total, preferably more than 12 C atoms in total which comprise either an acyl or an alkyl residue having each 1 to 5 carbon atoms. According to a preferred embodiment, the carboxylic acid ester has the following formula (I)

$$R^1COO\text{-}R^2$$

wherein (i) $R^1CO$ represents an acyl residue having 6 to 28 carbon atoms, and $R^2$ represents an alkyl residue having 1 to 5 carbon atoms or (ii) R1CO represents an acyl residue having 1 to 5 carbon atoms, and R2 represents an alkyl residue having 6 to 28 carbon atoms.

In line with option (i), the acyl residue may be saturated or unsaturated (*e.g.* 1,2,3 double bonds), the saturated embodiments being preferred. The acyl residue, preferably the saturated acyl residue may be branched and is optionally substituted, although this is not preferred. Similarly, the alkyl residue may be branched as in isopropyl and/or substituted. The acyl residue preferably has 12 to 22 carbon atoms, in particular, 14 to 20 carbon atoms. The alkyl residue preferably has 1 to 3 carbon atoms as in methyl, ethyl or (iso)propyl.

In line with option (ii) the acyl residue may be branched and/or substituted for instance by hydroxy. According to one embodiment of option (ii), the acyl residue has 2 to 4 carbon atoms. The alkyl residue preferably has 12 to 22 carbon atoms, in particular 14 to 20 carbon atoms. It may be saturated or unsaturated (*e.g.* one, two or three double bonds), saturated embodiments being preferred. Moreover, the alkyl residue may also be branched and/or substituted.

In the following table, esters that can be used with preference as low polarity oils in the present invention are summarized.

| **INCI name** | CAS **No.** | **Trade Name** | **Provider** |
|---|---|---|---|
| | | | |
| Isopropyl Palmitate | 142-91-6 | Isopropyl Palmitate | Aldrich |
| Isopropyl Myristate | 110-27-0 | Isopropyl Myristate | Aldrich |
| Dicaprylyl Carbonate | 1680-31-5 | Cetiol CC | BASF |
| Caprylic/Capric Triglycerides | 73398-61-5 | Crodamol GTCC | Croda |
| Propylene Glycol Isostearate | 68171-38-0 | Cithrol PGMIS | Croda |
| Isopropyl Isostearate | 68171-33-5 | Crodamol IPIS | Croda |
| Isostearyl Isostearate | 41669-30-1 | Crodamol ISIS | Croda |
| $C_{12-15}$ Alkyl Benzoate | 68411-27-8 | Tegosoft TN | Evonik |
| Diheptyl Succinate, Capryloyl Glycerin/Sebacic Acid Copolymer | 1190099-88-7 15872-89-6 | Lexfeel N20 | Saci-Cfpa (Inolex) |

(continued)

| INCI name | CAS No. | Trade Name | Provider |
|---|---|---|---|
| Capryloyl Glycerin/Sebacic Acid Copolymer, Diheptyl Succinate | 1190099-88-7 15872-89-6 | Lexfeel N350 | Saci-Cfpa/Inolex |
| Coco Caprylate/Caprate | 95912-86-0 | BUB 810C | Stearinerie Dubois |
| Diisopropyl Adipate | 6938-94-9 | DUB DIPA | Stearinerie Dubois |
| Diisopropyl Sebacate | 7491-02-3 | DUB DIS | Stearinerie Dubois |
| Isocetyl Stearoyl Stearate | 97338-28-8 | DUB SSIC | Stearinerie Dubois |
| Triisostearin | 26942-95-0 | DUB TGIS | Stearinerie Dubois |
| Triethylhexanoin | 7360-38-5 | DUB TOCG | Stearinerie Dubois |
| Propanediol Dicaprylate | 56519-71-2 | DUB Zenoat | Stearinerie Dubois |
| Olus Oil | 68956-68-3 | Lipex Bassol C | Unipex/AAK |
| Cetyl Ethylhexanoate | 59130-69-7 | Schercemol CO Esters | Unipex/Lubrizol |

"Silicone oils" as meant herein refer to the group of monomeric, linear or cyclic silicones. According to a preferred embodiment of the present invention, cyclic silicones are used. They can generally be represented by the following structural element.

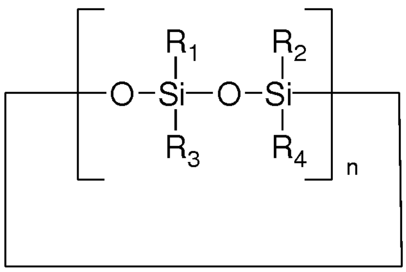

In the above formula, $R_1$ to $R_4$ represent identical or different alkyl radicals and/or aryl radicals. As will be appreciated, the meaning of $R_1$ to $R_4$ can vary between the n repeating units. In other words, the number of different radicals is not necessarily restricted to up to 4. n can in this case assume values from 3/2 to 20. Fractional values for n take into consideration that odd-numbered amounts of siloxyl groups can be present in the cycle.

In the present invention, the silicone oil can for example be selected from one of the following, or mixtures thereof: Phenyl trimethicone, dimethicone, phenyl dimethicone, cyclomethicone (for example hexamethylcyclotrisiloxane, octamethylcyclotetrasiloxane, cyclopentasiloxane, cyclohexasiloxane, and mixtures of these components), polydimethylsiloxane, poly-(methylphenylsiloxane), cetyl dimethicone, behenoxy dimethicone.

According to a preferred embodiment, the silicone oil is a cyclomethicone, in particular a mixture of cyclopentasiloxane and cyclohexasiloxane. A suitable cyclomethicone is for instance available from Dow Corning under the trade name Xiameter PMX-0345. The CAS No. of Xiameter PMX-0345 is 541-02-6. From the CAS No. it can be seen that Xiameter PMX-0345 is a permethylated cyclosiloxane, namely a mixture of permethylated cyclopentasiloxane and permethylated cyclohexasiloxane having the formulae $[(CH_3)_2SiO]_5$ and $[(CH_3)_2SiO]_6$. Consequently, the silicone oil is preferably $[(CH_3)_2SiO]_5$ and/or $[(CH_3)_2SiO]_6$.

2. Water

The second essential component (ii) of the cosmetic composition of, or for use in, the present invention is water. According to a preferred embodiment of the invention, the content of water, relative to the total mass of the cosmetic composition, is at least 40 % by mass, more preferably at least 50 % by mass, still more preferably it is at least 55 % by mass, even more preferably at least 60 % by mass, and most preferably at least 65 % by mass.

3. Emulsifier component

3.1 General

The third essential component of the cosmetic composition of, or for use in, the present invention is the emulsifier component (iii). The emulsifier component (iii) is contained in the cosmetic composition, in terms of the total mass of the cosmetic composition, in an amount of preferably 0.1 to 10 % by mass, more preferably in an amount of 0.25 to 7.5 % by mass, and most preferably in an amount of 0.5 to 5.0 % by mass.

3.2 Main emulsifier

The emulsifier component (iii) comprises a lipophilic non-ionic emulsifier. This is sometimes denoted as the main emulsifier herein. In the present patent application, a lipophilic (non-ionic) emulsifier is defined as an emulsifier having HLB < 6. The main emulsifier preferably has a HLB of from 3.5 to below 6, more preferably of from 4.5 to below 6.

Herein, HLB denotes the hydrophilic-lipophilic balance. As is well known to the skilled person, the HLB of a compound, such as an emulsifier, is a measure of the degree to which it is lipophilic or hydrophilic. To get a first idea of the HLB of a non-ionic compound at issue, Griffin's method as described in 1954 (Journal of the Society of Cosmetic Chemists (4), 249 - 56) can be used. According to Griffin's method, the HLB value is defined as follows:

$$HLB = 20 \times Mh/M$$

In the above formula Mh is the molecular mass of the hydrophilic portion of the molecule, and M is the molecular mass of the whole molecule, giving a result on a scale of 0 to 20. As will be appreciated, a HLB value of 0 corresponds to a completely lipophilic (or hydrophobic) molecule, and a value of 20 corresponds to a completely hydrophilic (or lipophobic) molecule.

The above method by Griffin can only provide a rough orientation of the HLB in that it provides a theoretical HLB. Therefore, the HLB is determined by way of experiment in the present application along the lines of the following methods.

In a first step, the approximate HLB value can be determined by the water dilution method, as follows. In a test tube filled with distilled water, the emulsifier to be tested is dissolved, either by heating if the product is solid, or by simple agitation if the product is liquid. After homogenization, the mixture is allowed to stand for some minutes, and the obtained dispersion is observed:

| **HLB** | 1-4 | 3-6 | 6-8 | 8-10 | 10-13 | 13+ |
|---|---|---|---|---|---|---|
| **Obtained Dispersion** | No dispersion | Faint dispersion | Milky dispersion after strong agitation | Stable milky dispersion | Translucent to clear dispersion | Clear dispersion |

This method is simple and quick. It allows giving an approximate HLB value.

Optionally having made the above approximation using the water dilution method, the HLB value is preferably determined in the present invention more precisely by way of the emulsification method, as follows. This experimental method consists in mixing the emulsifier to be tested in varying proportions with an emulsifier having a known HLB, and in using these mixtures to emulsify an oil with a known required HLB. This operation is carried out at room temperature if the product is liquid or at 75°C if the product is solid. The mixture giving the most stable emulsion has a HLB value of the emulsifier to be evaluated, using the following formula:

$$HLB_{required} = (HLB_A \times m_A + HLB_B \times m_B)/100$$

Example:

| Element | Percentage | | | | | | | | |
|---|---|---|---|---|---|---|---|---|---|
| **Emulsifier to be evaluated** | 4.5 | 4 | 3.5 | 3 | 2.5 | 2 | 1.5 | 1 | 0.5 |
| **Known emulsifier** | 0.5 | 1 | 1.5 | 2 | 2.5 | 3 | 3.5 | 4 | 4.5 |
| **Oil** | 15 | 15 | 15 | 15 | 15 | 15 | 15 | 15 | 15 |
| **Distilled water** | 80 | 80 | 80 | 80 | 80 | 80 | 80 | 80 | 80 |

Oil = Mineral oil
$HLB_{required}$ = 10
A = known emulsifier, Span 20
HLB = 8.7

If the most stable emulsion is the one with the 50/50 ratio of emulsifiers

$$10 = (8.7 \times 50 + HLB_B \times 50) / 100$$

Thus, $HLB_B$ = 11.3

According to a preferred embodiment, the main emulsifier is a carboxylic acid ester, in particular fatty acid ester, of polyalcohols. As is known to the skilled person, "fatty acids" is a generic term for aliphatic saturated carboxylic acids with almost exclusively unbranched carbon chains. The polyalcohols can be polyglycerol, glycerol, polyalkylene glycols (in particular polyethylene glycols (PEGs) and polypropylene glycols (PPGs)), sugars (in particular monosaccharides such as sucrose) and sorbitans. The PEGs and PPGs preferably have from 2 to 100, more preferably from 6 to 30 ethylene oxide (EO) and propylene oxide (PO) moieties, respectively. Amongst the above carboxylic acid esters, in particular fatty acid esters, the following groups are of particular interest:

1) Sorbitan esters

Examples of sorbitan esters that can be used as the main emulsifier in the present invention are shown in the following table.

| INCI/CTFA name | Trade name | Provider | HLB | Comments |
|---|---|---|---|---|
| | | | | |
| Sorbitan (mono)oleate | Span 80 | Croda | 4.3 | |
| Glycerol sorbitan oleostearate | Arlacel 481 | Croda | 4.5 | Contains hydrogenated castor oil, beeswax and stearic acid |
| Sorbitan isostearate | Arlacel 986 | Croda | 4.5 | Contains hydrogenated castor oil, cera alba and stearic acid |
| Sorbitan stearate and glucose cocoate | Arlacel 2121 | Croda | 3.4 | |
| Sorbitan oleate and polyglyceryl-3 polyricinoleate | Arlacel 1689 | Croda | 3.5 | |
| Sorbitan isostearate, polyglyceryl-3 polyricinoleate | Arlacel 1690 | Croda | 3.5 | |

2) Esters of glycerol and polyglycerol

As meant in this specification, a polyglycerol comprises at least two condensed glycerol moieties. Preferably, the number of condensed glycerol moieties in the esters is from 3 to 6.

Examples of main emulsifiers belonging to the above class of compounds are shown in the following table.

| INCI/CTFA name | Trade name | Provider | HLB | Comments |
|---|---|---|---|---|
| | | | | |
| Glyceryl oleate | Tegin O V | Evonik Industries AG Personal Care | 3.4 | |
| Glyceryl isostearate and polyglyceryl-3 oleate | Protegin W | Evonik Industries AG Personal Care | 4 | Contains further ingredients |
| Polyglyceryl 3/4 isostearate | Isolan GI 34 | Evonik Industries AG Personal Care | 5 | |
| Polyglyceryl 3 diisostearate | Cithrol 2621 Lameform TGI Cremophor GS 32 | Croda Henkel BASF | 4.7 | |
| Polyglyceryl 2 sesquiisostearate | Cithrol 2622 | Croda | 2.3 | |
| Polyglyceryl-2 dipolyhydroxystearate | Dehymuls PGPH | BASF | < 5 | |
| Polyglyceryl-3 oleate | Isolan GO 33 | Evonik Industries AG Personal Care | 5.5 | |
| Diisostearoyl polyglyceryl-3 dimer dilineolate | Isolan PDI | Evonik Industries AG Personal Care | 5 | |
| Polyglyceryl-3 polyricinoleate | Akoline PGPR | Unipex AAK | 4 | |
| Polyglyceryl-3 diisostearate | Plurol Diisostearique | Gattefosse | 5 - 6 | |

3) Sucrose esters

An example of a main emulsifier belonging to the above class of compounds is sucrose distearate (CTFA name) marketed by Croda under the trade name Crodesta. It has a HLB of 3.

4) Esters with PEG group

Examples of main emulsifiers belonging to the above class of compounds are compiled in the following table.

| INCI/CTFA name | Trade name | Provider | HLB |
|---|---|---|---|
| | | | |
| PEG-30 dipolyhydroxystearate | Cithrol DPHS | Croda | 5 - 6 |
| PEG 1 glycerolsorbitanoleo-stearate | Arlacel 581 | ICI | 5 |
| PEG-22/dodecyl glycol copolymer | Elfacos ST 37 | AkzoNobel | 2.4 |
| Octyldodecanol, octyldodecyl xyloside, PEG-30 dipolyhydroxystearate | Easynov | Seppic | <6 |

Further, silicone emulsifiers can be used as the main emulsifier in the present invention. In particular, copolymers of polyethers and polysiloxanes are useful. For instance, Bis-PEG/PPG-14/14 Dimethicone and cyclopentasiloxane (INCI/CTFA name) marketed by Evonik Industries under the trade name Abil EM 97 and Polyglyceryl-4-Isostearate and Cetyl PEG/PPG-10/1 Dimethicone and Hexyl Laurate (INCI/CTFA name) marketed by Evonik Industries AG Personal Care under the trade name Abil WE 09 can be used.

According to a preferred embodiment of the invention, the main emulsifier is selected from the group consisting of fatty acid esters of polyoxyalkylenes, in particular PEG, and fatty acid esters of glycerols and polyglycerols. Most preferred in the present invention are fatty acid esters, in particular diesters, of polyglycerols, especially those having from 3 to 6 condensed glycerol moieties in the molecule.

According to the most preferred embodiment, the main emulsifier is a fatty acid ester of polyglycerol, especially of a polyglycerol having from 3 to 6 condensed glycerol moieties in the molecule.

3.3 Co-emulsifier

According to a preferred embodiment of the invention, the emulsifier component (iii) further comprises a hydrophilic emulsifier. The further hydrophilic emulsifier is sometimes denoted for simplicity as co-emulsifier herein. In the present application, a hydrophilic emulsifier is defined as an emulsifier having a HLB value of more than 6, preferably of at least 8. Thereby, the HLB value is preferably determined by the emulsification method as described above, optionally after obtaining an approximate HLB value by way of the water dilution method as described above.

Both, in the case of the main emulsifier and in the case of the co-emulsifier, a mixture of more than one emulsifier can be used. However, according to a preferred embodiment, only a single main emulsifier and a single co-emulsifier are used in the present invention.

If at least one hydrophilic co-emulsifier is present together with the lipophilic non-ionic main emulsifier in the emulsifier component (iii), the mass ratio (co-emulsifier:main emulsifier) preferably is at least 1, more preferably at least 3, still more preferably at least 5, and most preferably at least 7.

As the co-emulsifier, non-ionic, anionic, cationic and amphoteric or zwitterionic emulsifier can be used, with non-ionic emulsifiers being preferred. Such emulsifiers are known from the art and some are, for instance, listed in Kirk-Othmer, Encyclopedia of Chemical Technology, 3rd Edition, 1979 (Wiley), Vol. 22, pp. 333 - 432; in EP 2 198 837 A1 (paragraph [0027] to [0068]); EP 2 111 840 A1 (paragraphs [0008] to [0013]); and EP 1 621 230 A1 (paragraphs [0064] to [0122]).

More specifically, anionic surfactants can be selected from the group consisting of protein and polypeptide derivatives; carboxylates such as amido ether carboxylates; salts of polyethoxylated carboxylic acids and salts of fatty acids having a $C_{16}$ - $C_{22}$ alkyl chain neutralized with an organic or mineral base, which constitute soaps (alkali soaps or amino soaps); amino acid derivatives, such as alkyl sarcosinates, alaninates, glutamates, aspartates, glycinates and citrates; taurates; sulfates, such as sodium, ammonium, potassium, triethanolamine (TEA) alkyl sulfates and alkyl ether sulfates of sodium, ammonium, magnesium, potassium and TEA; sulfonates, such as paraffine and olein sulfonates, alkyl sulfoacetates and isethionates; anionic derivatives of alkyl polyglucosides, such as sulfosuccinates, citrates, tartrates, carbonates and ethers of glycerol ethers obtained starting from alkyl glucosides; and phosphates, such as sodium alkyl phosphates and sodium alkyl ether phosphates.

Cationic emulsifiers may be quaternary ammonium compounds, alkyl pyridinium chlorides, alkyl ammonium saccharinates, and aminoxides at a pH below 6.5.

The amphoteric or zwitterionic emulsifiers may be betaine derivatives such as alkyl betaines and alkyl aminobetaines; sultaines; alkyl polyaminocarboxylates; alkyl amphoacetates; and imidazole derivatives.

As mentioned above, the co-emulsifier of, or for use in, the present invention is preferably a non-ionic emulsifier. The non-ionic co-emulsifier is preferably selected from the following groups (1) to (6) and mixtures thereof:

(1) Ethylenoxide (EO) or propylenoxide (PO) adducts of fatty alcohols having from 8 to 24 C atoms (in particular 12 to 22 C atoms), (C8-C15 alkyl)-phenol or polyols, containing 2 to 50 mol ethylenoxy and/or 0 to 5 mol propylenoxy units.

(2) Mono- or diesters (or mixtures thereof) derived from alkylene oxides and polyalkylene oxides, in particular PEG and PPG, glycerol, polyglycerol, poly-, oligo- or monosaccharides, sugar alcohols or sugar alcohol anhydrides (such as sorbitan), and linear or branched, saturated or unsaturated carboxylic acids having preferably 6 to 22 carbon atoms. These esters may also be ethoxylated (→ ethylene oxides (EO) units), *e.g.* polysorbate monolaurate + 20 EO or polysorbate monooleate + 20 EO. If the ester is to be liquid, the carboxylic acid can often be selected from short chain saturated carboxylic acid, *e.g.* as in sorbitan monolaurate or from carboxylic acids having at least one unsaturated fatty acid, as in sorbitan sesquioleate. More preferably, component (2) represents $C_{12/18}$ carboxylic acid monoesters and diesters of addition products of 1 to 50 mol ethylene oxide onto glycerol;

(3) C12 to 18 carboxylic acid, in particular fatty acid monoesters and diesters of addition products of 1 to 50 mol ethylene oxide onto glycerol.

(4) An alkyl mono-, oligo- and/or polyglycoside, preferably an alkyl mono- or oligo glycoside having 6 to 22 carbon atoms in the alkyl group and alkoxylated, preferably ethoxylated derivates thereof.

An alkyl(mono or oligo)glycoside is a nonionic surfactant wherein at least one hydroxy group (typically the C1 hydroxy of the first glycosyl) of a (mono or oligo)glycoside is linked via at least one ether bond (or ethyleneoxi and/or propyleneoxi units) with an alkyl group-bearing unit (preferably 6 to 28 C atoms in total) . The alkyl(mono or oligo) glycoside preferably has the following generic structure (II):

$$R^2O(C_nH_{2n}O)_t\ (\text{glycosyl})_x \qquad (II)$$

wherein $R^2$ is selected from the group consisting of alkyl, alkylphenyl, hydroxyalkyl, hydroxyalkylphenyl, and mixtures thereof in which the alkyl group contains from 6 to 22 carbon atoms, in particular from 8 to 16 carbon (*e.g.* 10 to 14 carbon atoms); n is 2 or 3, preferably 2, t is from 0 to about 10, preferably 0; x is at least 1, preferably from 1.1 to 5, more preferably 1.1 to 1.6, in particular 1.1 to 1.4, and "glycosyl" is a monosaccharide. The x value is to be understood as the average content of monosaccharide units (oligomerization degree).

The production of alkyl(oligo)glycoside useful in the present invention is known from the prior art and described, for instance in US-4,011,389, US-3,598,865, US-3,721,633, US- 3,772,269, US-3,640,998, US-3,839,318, or US-4,223,129.

To prepare these compounds, the alcohol or alkyl-polyethoxy alcohol is typically first formed and then reacted with the (oligo)glycosyl unit to form the (oligo)glycoside (attachment at the 1-position). The glycosyl units can be attached between the C1 position of further glycosyl(s) and the alkyl-group-bearing glycosyl unit's 2-, 3-, 4- and/or 6-position, preferably 6-position.

Preferred starting alcohols $R^2OH$ are primary linear alcohols or primary alcohols having a 2-methyl branch. Preferred alkyl residues $R^2$ are for instance 1-octyl, 1-decyl, 1-lauryl, 1-myristyl, 1-cetyl, and 1-stearyl, the use of 1-octyl, 1-decyl, 1-lauryl, and 1-myristyl being particularly preferred.

Alkyl(oligo)glycosides useful in the invention may contain only one specific alkyl residue. Usually, the starting alcohols are produced from natural fats, oils or mineral oils. In this case, the starting alcohols represent mixtures of various alkyl residues.

In four specific (preferred) embodiments alkyl(oligo)-glycosides are used, wherein $R^2$ consists essentially of $C_8$ and $C_{10}$ alkyl groups, $C_{12}$ and $C_{14}$ alkyl groups, $C_8$ to $C_{16}$ alkyl groups, or $C_{12}$ to $C_{16}$ alkyl groups.

It is possible to use as sugar residue "(glycosyl)$_x$" any mono- or oligosaccharide. Usually, sugars having 5 or 6 carbon atoms as well as the corresponding oligosaccharides are used. Such sugars include, for instance glucose, fructose, galactose, arabinose, ribose, xylose, lyxose, allose, altrose, mannose, gulose, idose, talose and sucrose. It is preferred to use glucose, fructose, galactose, arabinose, sucrose as well as their oligosaccharides, (oligo)glucose being particularly preferred.

In a preferred embodiment "laurylglucoside", a $C_{12}$-$C_{16}$ fatty alcohol-glucoside (x = 1.4), which can be obtained from BASF under the tradename Plantacare®, is used.

(5) Partial esters based on linear, branched, unsaturated or saturated $C_{6\text{-}22}$ carboxylic acids, ricinoleic acid and 12-hydroystearic acid and glycerol, polyglycerol, pentaerythritol, dipentaerythritol, sugar alcohols, alkyl glucosides and polyglucosides; or

(6) Polysiloxane/polyalkyl/polyether copolymers and corresponding derivatives.

According to a most preferred embodiment, the hydrophilic co-emulsifier is a monoester of a fatty acid having 6 to 22 carbon atoms with PEG, in particular PEG having 4 - 10 EO moieties.

In a particularly preferred embodiment of the cosmetic composition of, or for use in, the present invention, the emulsifier component (iii) comprises as main emulsifier a fatty acid diester of polyglycerol, especially of polyglycerol having from 3 to 6 condensed glycerol moieties in the molecule, and, as co-emulsifer, a monoester of a saturated carboxylic acid (fatty acid) having 6 to 22 carbon atoms with PEG having from 4 to 10 EO moieties.

4. Optional further Components

The cosmetic composition of, or for use in, the present invention optionally comprises further components.

Namely, the cosmetic composition optionally comprises, preferably in an amount of, expressed in terms of the total mass of the cosmetic composition, from 0.2 to 3.5 % by mass, more preferably of from 1.0 to 3.0 % by mass, of a hydrating agent. The hydrating agent can also be denoted as an emollient. The optional hydrating agent preferably is a polyhydroxy compound, which is understood to be an organic compound having at least two hydroxy groups and which preferably consists only of carbon, hydrogen, oxygen and nitrogen, in particular only of C, H and O. It is further desirable that the hydrating agent is not ionic.

The hydrating agent preferably has a liquid consistency, even though it is possible to use a minor amount of a

solid, low melting point hydrating agent depending on the desired viscosity and penetration behaviour of the cosmetic composition.

If liquid hydrating agents are to be employed, the molecular weight (weight average) preferably is less than 1,000, more preferably less than 800, and in particular not more than 600.

Examples of suitable hydrating agents include: amino acids, pyrrolidone, carboxylic acid, lactic acid and salts thereof, lactitol, urea and urea derivatives, uric acid, glucosamine, creatinine, cleavage products of collagen, chitosan or chitosan salts/derivatives and, in particular, polyols and polyol derivatives (for example glycerol, diglycerol, triglycerol, polyalkylene glycols, *e.g.* polypropylene glycol, butylene glycol, 1,2,6-hexantriol, polyethylene glycols, for instance polyethylene glycol having a weight average molecular weight of from about 200 to 600); neopentyl alcohols such as pentaerythritol or neopentyl glycol; sugar alcohols such as threitol, erythritol, adonitol (ribitol), arabitol, xylitol, dulcitol, maltitol, mannitol, inositol and sorbitol, carbohydrates such as D (+)- glucose, D (+)- fructose, D (+)-galactose, D (+)-mannose, L-gulose, saccharose, galactose, maltose, polyglycerols, polyoxypropylene adducts of glycerol, methoxypolyethylene glycol, polyethylene glycol ethers of sugar alcohols, such as sorbitol, polyethylene glycol ethers of glycerol, ethoxylated sorbitol (Sorbeth-6, Sorbeth-20, Sorbeth-30, Sorbeth-40), honey and hydrogenated honey, hydrogenated starch hydrolyzates and mixtures of hydrogenated wheat protein and PEG-20-acetate copolymer, and combinations thereof.

Preferred hydrating agents are butylene glycol, glycerol, diglycerol and triglycerol, and particularly preferred is butylene glycol.

Further, the cosmetic composition optionally comprises one or more preservatives, preferably in a total amount, expressed relative to the total mass of the cosmetic composition, in a range of 0.1 to 1.0, more preferably 0.2 to 0.4 % by mass. The preservative is preferably not derived from 4-hydroxy benzoic acid (paraben). Examples of useful preservatives are chlorphenesin, polyaminopropyl biguanide, methylisothiazolinone and dehydroacetic acid.

Another optional ingredient of the cosmetic composition are cosmetic agents, preferably from natural sources (plant extracts), having for instance a skin-soothing and/or antiphlogistic (reduction of skin irritation) effect such as allantoin; aloe vera extract; cornflower extract; cotton oil extract; cotton seed extract; aloe barbadensis extract; chamomile extract containing azulene and $\alpha$-bisabolol; echinacea; dragosantol; panthenol; liquorice root extract containing 18-glycyrrhetinic acid; lime tree extract containing quercetin and/or glyco-rutin; marigold (calendula oil); urea; phytosterols, optionally ethoxylated (available from Henkel under the tradename "Generol"); chitosan (acetylated chitin); anthocyanidins; gingko leaf extract containing quercetin and rutin; horse chestnut containing quercetin and campherol; vitamins or provitamins such as provitamin B5 or Vitamin E; avocado oil; birch extract; arnica; extract of rose of Sharon or St. John's wort; teatree oil; cucumber, hops, or hamamelis extracts or ingredients, ethoxylated quaternary amines (itching inhibiter useful for lotioned toilet papers). The use of panthenol, corflower extract, and cotton oil or seed extract is preferred. As will be appreciated from the above list, the cosmetic agents may be oils which may have a polarity of below 20 mN/m. The cosmetic agents can be present in an amount, in terms of the total mass of the cosmetic composition, of preferably 0.1 to 4.0, more preferably 0.2 to 3.0, still more preferably 0.3 to 1.0 and most preferably 0.1 to 0.3 % by mass.

Further optional components of the cosmetic compositions are salts, in particular inorganic salts, such as sodium chloride and disodium phosphate. The amount of the, preferably inorganic, salts relative to the total mass of the cosmetic composition, is preferably 0.1 to 1.0, more preferably 0.1 to 0.5 % by mass.

Still further, the cosmetic composition may contain one or more perfumes in an amount of preferably 0.1 to 0.3 % by mass, in terms of the total weight of the cosmetic composition.

Another optional component is one or more chelating agents, such as EDTA 2Na and EDTA 4Na, preferably in an amount, relative to the total mass of the cosmetic composition, of 0.1 to 0.15 % by mass.

Still further, the cosmetic composition can optionally contain a pH adjuster, such as citric acid, in an amount of from 0.01 to 1.0, more preferably 0.02 to 0.5 % by mass, still more preferably 0.03 to 0.1 % by mass, in terms of the total mass of the cosmetic composition.

5. Manufacturing the W/O emulsion

The W/O emulsion of, or for use in, the present invention can be prepared according to known methods. Generally, the water, *i.e.* aqueous, phase and the oil phase are slowly and progressively incorporated one into the other. Preferably, the aqueous phase is incorporated into the oil phase.

In the present invention, the aqueous phase comprises, apart from water, the optional hydrating agent(s), and optional water-soluble or water-dispersible additives, such as hydrophilic cosmetic agents, preservatives, inorganic salts, chelating agents and pH adjusters. These components of the aqueous phase are separately mixed. Similarly, the oil phase comprising the oil component (i) and the emulsifier component (iii), optionally perfume, and optionally lipophilic cosmetic agents such as plant extracts are mixed beforehand.

There are several variants of incorporating the aqueous phase into the oil phase:

- Hot-hot-process: Both, the aqueous phase and the oil phase are heated to 80 - 95°C, preferably 85 - 90°C;
- Hot-cold-process: Only the oil phase is heated, for instance to temperatures of 80 - 95°C, preferably 85 - 90°C, with the aqueous phase being cold, for instance at room temperature.
- Cold-cold-process: Both, the aqueous and the oil phase are cold, for instance at room temperature (23°C).

In a preferred embodiment, the W/O emulsion of, or for use in, the present invention is prepared by a cold-cold process as follows:
To prepare the aqueous phase, water is weighed, and may be heated to 50 °C, prior to admixing under stirring (for this, manual stirring is acceptable) or vigorous stirring (for instance at 700-900 rpm, preferably using an Ystral-type mixer) the further components of the aqueous phase. The addition of the components of the aqueous phase may be done stepwise, with homogeneous mixing, e.g. for 10 minutes, for instance at 700-900 rpm (preferably using an Ystral-type mixer) in between. Once all components of the aqueous phase, except for the pH adjuster(s), have been combined, the mixture is mixed homogeneously, e.g. for 20-30 min, for instance at 700-900 rpm (preferably using an Ystral-type mixer). All of this preferably occurs at temperatures of from room temperature (e.g. 23 °C) to 35 °C. Subsequently, the homogeneity of the obtained aqueous phase is checked: The aqueous phase should be limpid and colorless. Finally, the pH value of the aqueous phase may be adjusted using the pH adjuster(s), with the preferred pH value being around 6.0.

For preparing the oil phase, the components of the oil phase are mixed homogeneously. Preferably, this is done at 700-900 rpm, for instance using an Ystral-type mixer. Typical mixing times are 30 min. The preparation of the oil phase preferably takes place at room temperature (e.g. 23 °C). Finally, the homogeneity of the obtained oil phase is checked: The oil phase should be limpid to slightly turbid, and colorless.

The thus-obtained aqueous phase is slowly added to the thus obtained oil phase under very vigorous stirring, for instance at 5,000 to 6,000 rpm, using a suitable dispersion device such as supraton or stator-rotor homogenizers, in particular of the Ultra Turrax or Ystral type. This is done with the aqueous phase and the oil phase each preferably at a maximum of 30 °C (cold-cold-process). After continued emulsification under these conditions (preferably for 3-5 min), the stirring speed is reduced (for instance to 700-900 rpm) and the mixing is continued, preferably for another 20 minutes, to give the final W/O emulsion. Then, the pH of the final W/O emulsion may be controlled, which is preferably around 6.5.

6. Cosmetic composition

In the present invention, the viscosity of the cosmetic composition in the form of a W/O emulsion can be as low as 300 mPa·s or less, it is more preferably ≤ 200 mPa·s, still more preferably < 180 mPa·s, and most preferably it is < 160 mPa·s.

In this application, the viscosity of the cosmetic composition can be measured at 25°C using the viscosimeter Brookfield LV-DV-I from BROOKFIELD ENGINEERING LABORATORIES, INC. Depending from the expected range of viscosities, the following spindles (sp) and rotational speeds (v) are used:

| | | |
|---|---|---|
| - sp 1 / v 100 rpm | for | x < 0.1 Pa s |
| - sp 2 / v 100 rpm | for | 0.1 Pa s < x < 1 Pa s |
| - sp 3 / v 100 rpm | for | 1 Pa s < x < 6 Pa s |
| - sp 4 / v 100 rpm | for | x > 6 Pa s. |

As the present inventors found, for a given oil component (i) and a given emulsifier component (iii), the higher the amount of water, the more viscous is the W/O emulsion, and the higher the amount of the oil component, the less viscous is the W/O emulsion. Furthermore, the inventors found that the viscosity of the cosmetic composition can be adjusted by the type of the oil component (i) and the emulsifier component (iii). For instance, a cosmetic composition comprising, as an oil component (i), a mixture of a $C_{12}$ - $C_{16}$ alkane and a cyclic silicone, and as an emulsifier component (iii) a fatty acid diester of polyglycerol, especially of polyglycerol having from 3 to 6 condensed glycerol moieties in the molecule, together with a monoester of a branched unsaturated carboxylic acid (fatty acid) having 6 to 22 carbon atoms with PEG having from 4 to 10 EO moieties can have a viscosity as low as about 150 mPa·s.

7. Applicator for cosmetic purpose

In the present invention, the cosmetic composition is coated onto or impregnated into an applicator for cosmetic purpose. Thereby, the expression "coated onto" is meant to refer to a situation where the cosmetic composition is located primarily on the surface of the applicator, whereas the "impregnated into" indicates that the applicator is soaked through by the cosmetic composition. As will be appreciated, the applicator for cosmetic purpose may be coated and impregnated

with the cosmetic composition at the same time so that the cosmetic composition is distributed homogenously throughout the applicator. This is a preferred embodiment of the invention.

In the present invention, the applicator for cosmetic purpose preferably is a non-woven substrate, especially a wipe or pad. As is known to the skilled person, non-wovens are materials made of filaments and/or fibers. The non-woven substrate for use as the applicator for cosmetic purpose in the present invention is preferably a fibrous substrate, *i.e.* a substrate based on fibers. As meant herein, a fibrous substrate may without limitation comprise, expressed in terms of the mass of the substrate, at least 70 % by mass, especially at least 90 % by mass of fibers. The fibers in the non-woven substrate constituting the applicator are not specifically limited in kind and can be synthetic, artificial or natural fibers. According to a preferred embodiment, the applicator for cosmetic purpose is a wipe or a pad, in particular a pad, constituted of at least 50 %, more preferably at least 95 %, most preferably 100 % cellulosic fibers, in particular cotton fibers. The applicator for cosmetic purpose can preferably absorb from 2 to 6, more preferably from 3 to 5 g/g of the cosmetic composition.

According to a particularly preferred embodiment, the applicator is a pad having the following characteristics in combination:

- It is constituted of at least 50 %, preferably at least 95%, more preferably 100 % by weight of cotton fibers, expressed in terms of the pad without cosmetic composition coated and/or impregnated therein.
- It has a thickness, prior to coating and/or impregnating the cosmetic composition, of at least 1.2 mm, more preferably of at least 1.8 mm, and most preferably between 1.9 and 2.1 mm. Herein, the thickness is measured in accordance with the norm Edana 30-5-99 for normal nonwovens. See paragraph 5-2 of the measurement method presented in the norm. The measurement is carried out under a pressure of 0.5 kPa.
- It has a grammage, i.e. mass per unit area, prior to coating and/or impregnating the cosmetic composition, of between 100 and 220 g/m$^2$, preferably between 150 and 200 g/m$^2$, and most preferably between 170 and 190 g/m$^2$. Herein, the grammage is measured in accordance with the norm Edana 40.3-90 (February 99) after conditioning the sample for 24 h at 23 °C and 50 % relative humidity (RH) and testing the sample at 23 °C and 50 % RH;
- It has a decohesion force, prior to coating and/or impregnating the cosmetic composition, of at least 1.5 N, preferably of at least 2.5 N, and most preferably of at least 3.5 N. The decohesion force is the force needed to separate two sides of the pad and is measured in accordance with the method as detailed in WO 2006/084991 A1, page 8, line 23 to page 9, line 7.
- It is a non-woven, in particular a wet-laid non-woven, especially a water jet wet-laid non-woven.

Pads having the above characteristics proved to be extremely well suited as the applicator for cosmetic purpose for use in the present invention. Suitable pads for use in the present invention are described in WO 2006/084991 A1. Cotton pads can be manufactured in accordance with the method as described in WO 94/17235 and US 5,849,647.

According to another embodiment of the invention, the applicator for cosmetic purpose is an air-laid non-woven, in particular an air-laid non-woven fibrous, i.e. fiber-based, substrate. An "air-laid" product as meant herein is prepared by way of air-laying. In line with the general understanding, air-laying is a process of depositing a web of air-borne fibers. That is, the fibers are carried and formed to the structure of the air-laid web by air. More specifically, air-laying is a non-woven web forming process that disperses fibers into a fast moving air stream and condenses them onto a moving screen by means of pressure or vacuum. Sometimes, air-laying is referred to as an aerodynamic web forming method.

The fibers for use in the air-laying as outlined above are not specifically limited in kind and synthetic, artificial and synthetic fibers may equally be used. Cellulosic fibers, in particular cellulose pulp fibers, especially cellulose fluff pulp fibers, and thermo-bonding fibers are used with preference.

Air-laying gives an air-laid web of fibers that can be bonded by bonding techniques to stabilize the web of the air-laid fibers and in particular to provide fabric integrity. The air-laid web of fibers after bonding is referred to as air-laid non-woven herein. The preferred bonding technique comprises applying a binder to the web with subsequent curing to give the air-laid non-woven. Without limitation, the binder can be a synthetic binder, such as a latex, and a natural binder, such as starch. The synthetic binder can be an ethylene/vinyl acetate copolymer composition. The amount of the binder in the final product, *i.e.* the air-laid non-woven, will be chosen by the skilled person according to the needs, and is preferably in the range of from 5 to 30 % by mass, more preferably from 10 to 20 % by mass, in terms of mass of the air-laid non-woven.

Within the above embodiment of the present invention, *i.e.* wherein the applicator for cosmetic purpose is an air-laid non-woven, the fibers are preferably cellulose fluff pulp fibers, and to bond the web of the air-laid fibers, a synthetic binder is preferably applied to the web with subsequently curing to give the air-laid non-woven, especially the air-laid non-woven fibrous substrate for use according to this embodiment of the present invention. Consequently, the air-laid non-woven, especially the air-laid non-woven fibrous substrate, constituting the applicator for cosmetic purpose according to this embodiment of the invention preferably comprises, in an amount of 5 to 30 % by mass with respect to the mass

of the air-laid non-woven, a synthetic binder, namely a latex, with the remainder constituted of cellulose fluff pulp fibers.

While current technologies such as spraying or soaking can be used for providing the applicator for cosmetic purpose with the cosmetic composition (here by coating and/or impregnating), to the surprise of the inventors, the cosmetic composition can be impregnated homogenously into a pile of applicators, as follows. Owing to its high fluidity, the cosmetic composition of, or for use in, the present invention can be coated and impregnated into a pile of applicators, in particular pads that are stacked on one another, by means of a single injection of the cosmetic composition at the top of the pile. Unlike with make-up removing cosmetic compositions of the prior art, the impregnation can be carried out in a homogenous manner even in this case. The homogenous impregnation of several applicators stacked in a pile by a single injection at the top of the pile, which is a most economical process, works particularly well when the viscosity of the cosmetic composition is < 200 mPa·s, preferably < 180 mPa·s and more preferably < 160 mPa·s. For instance, a stack of 5 to 40 pads, more preferably 10 to 30 pads can be impregnated in this way. Such methods for impregnating applicators for cosmetic purpose, such as pads, are described in US 2010/0297191 A1.

Upon use, the applicator for cosmetic purpose having the cosmetic composition in the form of a W/O emulsion coated and/or impregnated therein, is wiped over those parts of the skin from which make-up, in particular waterproof make-up is to be removed, in particular the face and the eyes. Upon the slight pressure exerted upon wiping, the cosmetic composition is released towards the skin.

The present invention will be further described by way of the following examples, which of course must not be construed in a limiting sense.

EXAMPLES

Example (in accordance with the invention)

A cosmetic composition in the form of a W/O emulsion comprising the following components, with the amounts expressed in % by mass in terms of the mass of the entire cosmetic composition, was prepared:

- 17.00 % isododecane, and 8.00 % cyclomethicone, namely, a mixture of decamethylcyclopentasiloxane and dodecamethylcyclohexasiloxane (available from Dow Corning under the trade name Xiameter PMX-0345), constituting the oil component (i);
- 0.50 % polyglyceryl-3 diisostearate (INCI name) available from Gattefosse under the trade name Plurol Diisostearique constituting a lipophilic non-ionic emulsifier (HLB 5 - 6), and 4.50 % of PEG-6 isostearate (INCI name) available from Gattefosse under the trade name Olepal Isostearique representing a hydrophilic non-ionic co-emulsifier (HLB = 9);
- 2.00 % hydrating agent;
- 0.10 % chelating agents;
- 0.208 % non-paraben preservatives;
- 0.35 % cosmetic agents;
- 0.20 % perfume;
- 0.42 % inorganic salts;
- 0.05 % pH adjusters; and
- water ad 100 %.

The viscosity of the above W/O emulsion, measured in accordance with the method as described in the general description, was about 150 mPa·s.

Comparative Example

A cosmetic composition, which is an O/W emulsion and comprises, in % by mass in terms of the total mass of the cosmetic composition, the following components, was prepared:

- 6.00 % mineral oil, 3.00 % isohexadecane and 2.00 % isopropyl palmitate forming an oil component;
- 4.00 % of hydrophilic non-ionic emulsifiers (with HLB of at least 10), namely 3.00 % of a mixture of glyceryl stearate, Ceteareth-20, Ceteareth-12, cetearyl alcohol and cetyl palmitate (available from BASF under the trade name Emulgade SE-PF; HLB > 10), and 1.00% of Ceteareth-20 (available from BASF under the trade name Eumulgin B2; HLB = 15.5);
- 3.00 % hydrating agent;
- 0.10 % chelating agent;
- 0.50 % non-paraben preservatives;

- 3.35 % cosmetic agents;
- 0.10 % perfume; and
- Water ad 100%.

Test for removing make-up

To compare the capability of the cosmetic compositions of the Example and Comparative Example to remove make-up, the following spectrophotometric test was carried out using round cotton pads, i.e. pads consisting of cotton fibers only, having a weight of 0.46 g and a grammage of 180 $g/m^2$, impregnated at 4.5 g/g with the cosmetic compositions of the Example and Comparative Example. A Konica Minolta CM-2600d spectrophotometer was employed in the test using the software SPECTRAMAGIC NX.

The skin test area was the hairless inner side of the forearms of persons, which had not been treated with care cream for 12 h. Only one test was carried out per day. Blind values of the whiteness were obtained using the spectrophotometer for the target regions on the inner forearm. Then, the selected make-up products were applied to the target regions on the forearm back and forth twice on a length of 3 cm each, and allowed to dry for 5 minutes to give make-up patches in the target regions. The tested normal make-up products and waterproof make-up products were the following:

Normal make-up

- Lipstick classical (L'Oréal 712)
- Eye liner classical (L'Oréal Contour Khôl)
- Mascara classical (L'Oréal telescopic)
- Foundation classical (Gerney Instant Liss'result)

Waterproof make-up

- Mascara waterproof (Waterproof L'Oréal telescopic)
- Eye shadow stick waterproof (30 blue delicious waterproof Maybelline)

Then a value $\Delta E$ of the whiteness was measured in each of the target regions, taking into account the respective blind value obtained earlier. This gives the value $\Delta E1$. Subsequently, the cosmetic composition according to the Example or the Comparative Example was applied to the make-up patch by making 5 circular movements with the cotton pad impregnated with it. Then, possible residues from the cosmetic composition were removed by a move with the cotton pad from up to down. Then, the whiteness in the target areas was measured taking into account the blind value giving a value $\Delta E$, namely $\Delta E2$.

The efficacy of the make-up removal, expressed in %, is then calculated as follows:

$$\text{Efficacy (\%)} = 100 \times \frac{\Delta E1 - \Delta E2}{\Delta E1}$$

The principle underlying the above test is the difference between two colours $\Delta E$ as defined in the formula established by CIE in 1976, as follows:

$$\Delta E^{*} = \sqrt{((L_1 - L_2)^2 + (a_1 - a_2)^2 + (b_1 - b_2)^2},$$

wherein $L_1,a_1,b_1$ are the coordinates in the colour space CIE Lab of the first colour in comparison with the values $L_2,a_2,b_2$ of the second colour. Thus, the $\Delta E$ corresponds to the distance between two colours in the colour space.

The results of the above make-up removal efficacy test using the above-identified normal make-up products and waterproof make-up products are summarized in the following table:

| | Cosmetic composition of Example | Cosmetic composition of Comparative Example |
|---|---|---|
| **Normal make-up** | | |
| Lipstick classical | 92.62 | 82.69 |
| Eye liner classical | 91.64 | 83.57 |
| Mascara classical | 85.18 | 84.53 |
| Foundation classical | 84.48 | 80.96 |
| **Average for normal make-up** | **88.48** | **82.94** |
| **Waterproof make-up** | | |
| Mascara waterproof | 80.46 | 55.65 |
| Eye shadow stick waterproof | 83.49 | 76.92 |
| **Average for waterproof make-up** | **81.98** | **66.29** |

In the above table, the efficacy $\Delta E$ is expressed in %.

It can be seen from the above data that the inventive cosmetic composition is significantly more efficacious in removing make-up, particular in removing waterproof make-up, especially in removing waterproof mascara, than the Comparative Example based on a O/W emulsion like those on the market (Demak Up Sensitive) and those described for instance in EP 1 618 925 A1. The above largely improved efficacy was also impressively confirmed by a customer survey enrolling 254 customers. The customers had to compare the product of the Example with the one of the Comparative Example.

Furthermore, the W/O emulsion of the invention (as used in the Example), unlike the oil-based product on the market (*e.g.* "Quickies"), did hardly leave a greasy layer on the skin or eye or give an unpleasant oily sensation after use.

Meeting the conflicting targets of excellent removal efficacy for waterproof make-up and avoiding the formation of a greasy or oily film on the skin after application, the cosmetic composition of, or for use in, the present invention represents a remarkable improvement and therefore can be expected to be most attractive to customers.

EMBODIMENTS OF THE INVENTION

In the following, preferred embodiments of the present invention will be described by reference to items (1), (2), (3) etc.

(1) A use of a cosmetic composition for removing make-up,
wherein the cosmetic composition is in the form of a water-in-oil emulsion and comprises:

(i) an oil component having a polarity of at least 20 mN/m and comprising a non-polar oil having a polarity greater than 30 mN/m and a low polarity oil having a polarity in the range of from 20 to 30 mN/m with a mass ratio of nonpolar oil to low polarity oil being in the range of from 1 to 5,
(ii) water, and
(iii) an emulsifier component comprising a lipophilic non-ionic emulsifier; wherein the viscosity of the cosmetic composition is ≤ 300 mPa·s, preferably < 180 mPa·s; and

wherein the cosmetic composition is coated onto and/or impregnated into an applicator for cosmetic purpose, wherein the content of the oil component (i) is 22 to 40 % by mass, relative to the total mass of the cosmetic composition, preferably by wiping the make-up, which is especially waterproof make up, with the applicator.

(2) The use according to (1), wherein the applicator for cosmetic purpose is a wipe or pad and the cosmetic composition fulfills each of the following features (a) to (b):

(a) The content of water (ii) therein is least 40 % by mass, relative to the total mass of the cosmetic composition;
(b) The content of the emulsifier component (iii) therein is 0.1 to 10 % by mass, relative to the total mass of the

cosmetic composition.

(3) The use according to (1), wherein the applicator for cosmetic purpose is a wipe or pad and the cosmetic composition fulfills at least one, preferably all, of the following features (a) to (c):

(a) The content of the oil component (i) therein is 22 to 30 % by mass, relative to the total mass of the cosmetic composition;
(b) The content of water (ii) therein is least 65 % by mass, relative to the total mass of the cosmetic composition;
(c) The content of the emulsifier component (iii) therein is 3 to 7 % by mass, relative to the total mass of the cosmetic composition.

(4) The use according to any one of (1) to (3), wherein the viscosity of the cosmetic composition is < 180 mPa·s.

(5) The use according to any one of (1) to (3), wherein the viscosity of the cosmetic composition is < 160 mPa·s.

(6) The use according to at least one of (3), (4) and (5), wherein the oil component (i) is a mixture of hydrocarbon-based non-polar oil and cyclic silicone of the following formula:

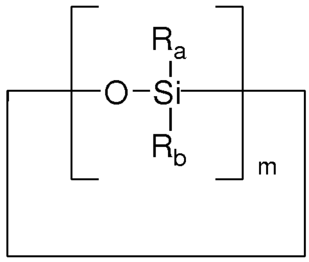
,

wherein $R_a$ and $R_b$ are each methyl or ethyl, preferably methyl, and m is 4 to 6, in particular 5 and/or 6.

(7) The use according to (6), wherein the mass ratio of the hydrocarbon-based non-polar oil and the cyclic silicone is in the range of 1 to 5, in particular 2 to 3, in the mixture of the oil component (i).

(8) The use according to at least one of (3), (4) and (5), wherein the oil component (i) is a mixture of $C_8$-$C_{32}$ alkane and cyclic silicone of the following formula:

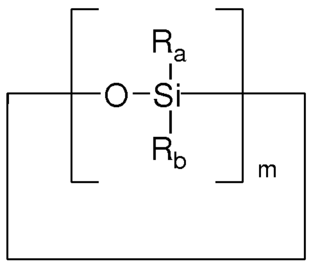
,

wherein $R_a$ and $R_b$ are each methyl, and m is 4 to 6, in particular 5 and/or 6.

(9) The use according to (8), wherein the mass ratio of the $C_8$-$C_{32}$ alkane and the cyclic silicone is in the range of 1 to 5, in particular 2 to 3, in the mixture of the oil component (i).

(10) The use according to at least one of (3) to (9), wherein the emulsifier component (iii) is a mixture of, as the lipophilic non-ionic emulsifier, a diester of a fatty acid with polyglycerol, and, as a hydrophilic non-ionic co-emulsifier, a monoester of a fatty acid with polyethylene glycol (PEG).

(11) The use according to (10), wherein each of the fatty acids is a saturated carboxylic acid having 6 to 22 carbon atoms, preferably having 16 to 20 carbon atoms.

(12) The use according to (10) or (11), wherein the polyglycerol has 3 to 6 condensed glycerol moieties in its molecule.

(13) The use according to any one of (10) to (12), wherein the polyethylene glycol has 4 to 10 ethylene oxide (EO) moieties in its molecule.

(14) The use according to any one of (10) to (13), wherein the ratio by weight of the lipophilic non-ionic emulsifier to the hydrophilic non-ionic co-emulsifier (lipophilic non-ionic emulsifier : hydrophilic non-ionic co-emulsifier) in the emulsifier component (iii) is 1 : ≥ 3, preferably 1 : ≥ 5.

(15) The use according to any one of (1) to (14), wherein the applicator for cosmetic purpose is a pad having the following properties in combination:

- It is a non-woven constituted of at least 95 % by mass of cotton fibres, expressed in terms of the mass of the pad;
- It has a thickness of between 1.9 and 2.1 mm;
- It has a grammage of between 170 and 190 $g/m^2$; and
- It has a decohesion force of at least 2.5 N.

(16) The use of a cosmetic composition for removing make-up according to (1), wherein:

the oil component (i) is contained in the cosmetic composition in an amount of 22 to 30 % by mass, relative to the total mass of the cosmetic composition, and is a mixture of $C_{12}$-$C_{16}$ alkane and cyclic silicone of the following formula:

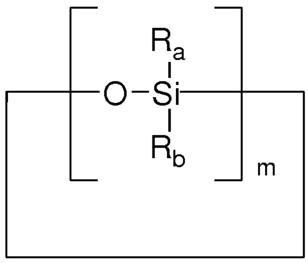

,

wherein $R_a$ and $R_b$ are each methyl, and m is 5 and/or 6, wherein the mass ratio of the $C_{12}$-$C_{16}$ alkane and the cyclic silicone in the mixture of the oil component (i)is in the range of 2 to 3;
water (ii) is contained in the cosmetic composition in an amount of at least 65 % by mass, relative to the total mass of the cosmetic composition;
the emulsifier component (iii) is contained in the cosmetic composition in an amount of 3 to 7 % by mass, relative to the total mass of the cosmetic composition, and is a mixture of, as the lipophilic non-ionic emulsifier, a diester of a saturated carboxylic acid having 16 to 20 carbon atoms (fatty acid) with polyglycerol having 3 to 6 condensed glycerol moieties in its molecule, and, as a hydrophilic non-ionic co-emulsifier, a monoester of a saturated carboxylic acid having 16 to 20 carbon atoms (fatty acid) with polyethylene glycol (PEG) having 4 to 10 ethylene oxide (EO) moieties in its molecule, wherein the mass ratio of the lipophilic non-ionic emulsifier to the hydrophilic non-ionic co-emulsifier (lipophilic non-ionic emulsifier : hydrophilic non-ionic co-emulsifier) in the emulsifier component (iii) is 1 : ≥ 5; and
wherein the viscosity of the cosmetic composition is < 180 mPa·s.

(17) The use according to (16), wherein the viscosity of the cosmetic composition is < 160 mPa·s.

(18) The use according to (16) or (17), wherein the applicator for cosmetic purpose is a wipe or pad.

(19) The use according to any one of (16) to (18), wherein the applicator for cosmetic purpose is a pad having the following properties in combination:

- It is a non-woven constituted of at least 95 % by mass of cotton fibres, expressed in terms of the mass of the pad;
- It has a thickness of between 1.9 and 2.1 mm;
- It has a grammage of between 170 and 190 $g/m^2$; and
- It has a decohesion force of at least 2.5 N.

(20) A method for removing make-up, especially waterproof make-up, comprising applying a cosmetic composition to the make-up,
wherein the cosmetic composition is in the form of a water-in-oil emulsion and comprises:

(i) an oil component having a polarity of at least 20 mN/m and comprising a non-polar oil having a polarity greater than 30 mN/m and a low polarity oil having a polarity in the range of from 20 to 30 mN/m with a mass ratio of nonpolar oil to low polarity oil being in the range of from 1 to 5,
(ii) water, and
(iii) an emulsifier component comprising a lipophilic non-ionic emulsifier; and

wherein the cosmetic composition is coated onto and/or impregnated into an applicator for cosmetic purpose, wherein the content of the oil component (i) is 22 to 40 % by mass, relative to the total mass of the cosmetic composition, preferably by wiping the make-up with the applicator.

(21) The method according to (20), wherein the applicator is a non-woven substrate.

(22) The method according to (21), wherein the non-woven substrate is a wipe or a pad.

(23) The method according to any one of (20) to (22), wherein the viscosity of the cosmetic composition is $\leq$ 300 mPa·s, preferably < 180 mPa·s.

(24) The method according to any one of (20) to (23), wherein the cosmetic composition has at least one, preferably all, of the following features (a) to (c):

(a) The content of the oil component (i) therein is 22 to 40 % by mass, relative to the total mass of the cosmetic composition;
(b) The content of water (ii) therein is least 40 % by mass, relative to the total mass of the cosmetic composition;
(c) The content of the emulsifier component (iii) therein is 0.1 to 10 % by mass, relative to the total mass of the cosmetic composition.

(25) The method according to any one of (20) to (24), wherein the oil component (i) comprises non-polar oil and/or low polarity oil.

(26) The method according to (25), wherein the non-polar oil is a $C_8$-$C_{32}$ hydrocarbon.

(27) The method according to (25) or (26), wherein the low polarity oil is selected from the group consisting of a silicone oil and a dialkylcarbonate.

(28) The method according to any one of (20) to (27), wherein the oil component (i) is a mixture of $C_{12}$-$C_{16}$ alkane and silicone oil, in particular a mixture of $C_{12}$-$C_{16}$ alkane and a cyclic silicone.

(29) The method according to any one of (20) to (28), wherein the emulsifier component (iii) further comprises a hydrophilic emulsifier, which preferably is a non-ionic emulsifier.

(30) The method according to any one of (20) to (29), wherein the emulsifier(s) of the emulsifier component (iii) is/are selected from the group consisting of fatty acid esters of polyoxyalkylenes and fatty acid esters of polyglycerols.

(31) The method according to (20), wherein the applicator for cosmetic purpose is a wipe or pad and the cosmetic composition fulfills at least one, preferably all, of the following features (a) to (c):

(a) The content of the oil component (i) therein is 22 to 30 % by mass, relative to the total mass of the cosmetic composition;
(b) The content of water (ii) therein is least 65 % by mass, relative to the total mass of the cosmetic composition;
(c) The content of the emulsifier component (iii) therein is 3 to 7 % by mass, relative to the total mass of the cosmetic composition.

(32) The method according to (31), wherein the viscosity of the cosmetic composition is < 180 mPa·s.

(33) The method according to (31), wherein the viscosity of the cosmetic composition is < 160 mPa·s.

(34) The method according to at least one of (31), (32) and (33), wherein the oil component (i) is a mixture of hydrocarbon-based non-polar oil and cyclic silicone of the following formula:

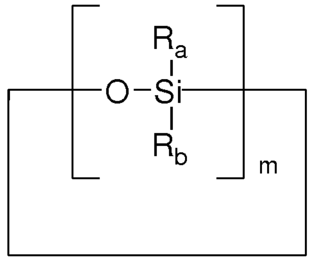

,

wherein $R_a$ and $R_b$ are each methyl or ethyl, preferably methyl, and m is 4 to 6, in particular 5 and/or 6.

(35) The method according to (34), wherein the mass ratio of the hydrocarbon-based non-polar oil and the cyclic silicone is in the range of 1 to 5, in particular 2 to 3, in the mixture of the oil component (i).

(36) The method according to at least one of (31), (32) and (33), wherein the oil component (i) is a mixture of $C_8$-$C_{32}$ alkane and cyclic silicone of the following formula:

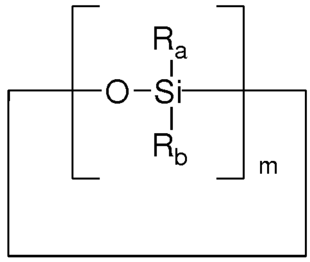

,

wherein $R_a$ and $R_b$ are each methyl, and m is 4 to 6, in particular 5 and/or 6.

(37) The method according to (36), wherein the mass ratio of the $C_8$-$C_{32}$ alkane and the cyclic silicone is in the range of 1 to 5, in particular 2 to 3, in the mixture of the oil component (i).

(38) The method according to at least one of (31) to (37), wherein the emulsifier component (iii) is a mixture of, as the lipophilic non-ionic emulsifier, a diester of a fatty acid with polyglycerol, and, as a hydrophilic non-ionic co-emulsifier, a monoester of a fatty acid with polyethylene glycol (PEG).

(39) The method according to (38), wherein each of the fatty acids is a saturated carboxylic acid having 6 to 22 carbon atoms, preferably having 16 to 20 carbon atoms.

(40) The method according to (38) or (39), wherein the polyglycerol has 3 to 6 condensed glycerol moieties in its molecule.

(41) The method according to any one of (38) to (40), wherein the polyethylene glycol has 4 to 10 ethylene oxide (EO) moieties in its molecule.

(42) The method according to any one of (38) to (41), wherein the ratio by weight of the lipophilic non-ionic emulsifier to the hydrophilic non-ionic co-emulsifier (lipophilic non-ionic emulsifier : hydrophilic non-ionic co-emulsifier) in the emulsifier component (iii) is 1 : ≥ 3, preferably 1 : ≥ 5.

(43) The method according to any one of (38) to (42), wherein the applicator for cosmetic purpose is a pad having the following properties in combination:

- It is a non-woven constituted of at least 95 % by mass of cotton fibres, expressed in terms of the mass of the pad;
- It has a thickness of between 1.9 and 2.1 mm;
- It has a grammage of between 170 and 190 $g/m^2$; and
- It has a decohesion force of at least 2.5 N.

(44) The method according to (20), wherein:

the oil component (i) is contained in the cosmetic composition in an amount of 22 to 30 % by mass, relative to the total mass of the cosmetic composition, and is a mixture of $C_{12}$-$C_{16}$ alkane and cyclic silicone of the following formula:

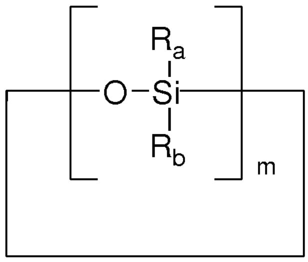

,

wherein $R_a$ and $R_b$ are each methyl, and m is 5 and/or 6, wherein the mass ratio of the $C_{12}$-$C_{16}$ alkane and the cyclic silicone in the mixture of the oil component (i)is in the range of 2 to 3;
water (ii) is contained in the cosmetic composition in an amount of at least 65 % by mass, relative to the total mass of the cosmetic composition;
the emulsifier component (iii) is contained in the cosmetic composition in an amount of 3 to 7 % by mass, relative to the total mass of the cosmetic composition, and is a mixture of, as the lipophilic non-ionic emulsifier, a diester of a saturated carboxylic acid having 16 to 20 carbon atoms (fatty acid) with polyglycerol having 3 to 6 condensed glycerol moieties in its molecule, and, as a hydrophilic non-ionic co-emulsifier, a monoester of a saturated carboxylic acid having 16 to 20 carbon atoms (fatty acid) with polyethylene glycol (PEG) having 4 to 10 ethylene oxide (EO) moieties in its molecule, wherein the mass ratio of the lipophilic non-ionic emulsifier to the hydrophilic non-ionic co-emulsifier (lipophilic non-ionic emulsifier : hydrophilic non-ionic co-emulsifier) in the emulsifier component (iii) is 1 : ≥ 5; and
wherein the viscosity of the cosmetic composition is < 180 mPa·s.

(45) The method according to (44), wherein the viscosity of the cosmetic composition is < 160 mPa·s.

(46) The method according to (44) or (45), wherein the applicator for cosmetic purpose is a wipe or pad.

(47) The method according to any one of (44) to (46), wherein the applicator for cosmetic purpose is a pad having the following properties in combination:

- It is a non-woven constituted of at least 95 % by mass of cotton fibres, expressed in terms of the mass of the pad;
- It has a thickness of between 1.9 and 2.1 mm;
- It has a grammage of between 170 and 190 $g/m^2$; and
- It has a decohesion force of at least 2.5 N.

(48) A cosmetic composition, in particular for removing make-up, especially waterproof make-up, which is in the form of a water-in-oil emulsion and comprises:

(i) an oil component which is a mixture of $C_{12}$-$C_{16}$ alkane and a cyclic silicone with a mass ratio of $C_{12}$-$C_{16}$ alkane to cyclic silicone being in the range of from 1 to 5;
(ii) water; and
(iii) an emulsifier component comprising a lipophilic non-ionic emulsifier, wherein the content of the oil component (i) is 22 to 40 % by mass, relative to the total mass of the cosmetic composition.

(49) The cosmetic composition of (48), which has at least one, preferably all, of the following features (a) to (b) :

(a) The content of water (ii) therein is least 40 % by mass, relative to the total mass of the cosmetic composition;
(b) The content of the emulsifier component (iii) therein is 0.1 to 10 % by mass, relative to the total mass of the cosmetic composition.

(50) The cosmetic composition of (48) or (49), which has a viscosity of ≤ 300 mPa·s, preferably < 180 mPa·s.

(51) The cosmetic composition of any one of (48) to (50), wherein the emulsifier component (iii) further comprises a hydrophilic emulsifier, which preferably is a non-ionic emulsifier.

(52) The cosmetic composition of any one of (48) to (51), wherein the emulsifier(s) of the emulsifier component (iii) is/are selected from the group consisting of fatty acid esters of polyoxyalkylenes and fatty acid esters of polyglycerols.

(53) The cosmetic composition of (48), which has at least one, preferably all, of the following features (a) to (c):

(a) The content of the oil component (i) therein is 22 to 30 % by mass, relative to the total mass of the cosmetic composition;
(b) The content of water (ii) therein is least 65 % by mass, relative to the total mass of the cosmetic composition;
(c) The content of the emulsifier component (iii) therein is 3 to 7 % by mass, relative to the total mass of the cosmetic composition.

(54) The cosmetic composition of (53), which has a viscosity of < 180 mPa·s.

(55) The cosmetic composition of (53), which has a viscosity of < 160 mPa·s.

(56) The cosmetic composition of any one of (53) to (55), wherein the cyclic silicone in the oil component (i) has the following formula:

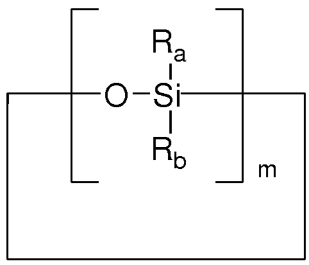

,

wherein $R_a$ and $R_b$ are each methyl or ethyl, preferably methyl, and m is 4 to 6, in particular 5 and/or 6.

(57) The cosmetic composition of any one of (53) to (55), wherein the cyclic silicone in the oil component (i) has the following formula:

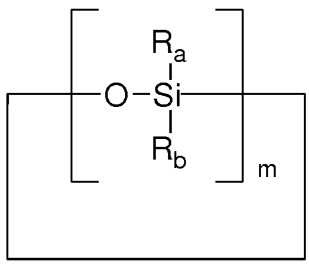

,

wherein $R_a$ and $R_b$ are each methyl, and m is 4 to 6, in particular 5 and/or 6.

(58) The cosmetic composition of (56) or (57), wherein the mass ratio of the $C_{12}$-$C_{16}$ alkane and the cyclic silicone is in the range of 1 to 5, in particular 2 to 3, in the mixture of the oil component (i).

(59) The cosmetic composition of (53) to (58), wherein the emulsifier component (iii) is a mixture of, as the lipophilic

non-ionic emulsifier, a diester of a fatty acid with polyglycerol, and, as a hydrophilic non-ionic co-emulsifier, a monoester of a fatty acid with polyethylene glycol (PEG).

(60) The cosmetic composition of (59), wherein each of the fatty acids is a saturated carboxylic acid having 6 to 22 carbon atoms, preferably having 16 to 20 carbon atoms.

(61) The cosmetic composition of (59) or (60), wherein the polyglycerol has 3 to 6 condensed glycerol moieties in its molecule.

(62) The cosmetic composition of any one of (59) to (61), wherein the polyethylene glycol has 4 to 10 ethylene oxide (EO) moieties in its molecule.

(63) The cosmetic composition of any one of (59) to (62), wherein the ratio by weight of the lipophilic non-ionic emulsifier to the hydrophilic non-ionic co-emulsifier (lipophilic non-ionic emulsifier : hydrophilic non-ionic co-emulsifier) in the emulsifier component (iii) is 1 : ≥ 3, preferably 1 : ≥ 5.

(64) The cosmetic composition of (48), wherein:

the oil component (i) is contained in an amount of 22 to 30 % by mass, relative to the total mass of the cosmetic composition, and the cyclic silicone is represented by the following formula:

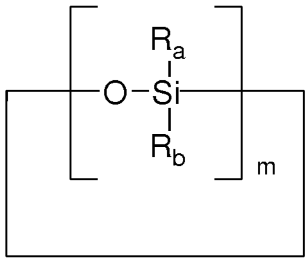

,

wherein $R_a$ and $R_b$ are each methyl, and m is 5 and/or 6, wherein the mass ratio of the $C_{12}$-$C_{16}$ alkane and the cyclic silicone in the mixture of the oil component (i) is 2 in the range from 2 to 3;
water (ii) is contained in an amount of at least 65 % by mass, relative to the total mass of the cosmetic composition;
the emulsifier component (iii) is contained in an amount of 3 to 7 % by mass, relative to the total mass of the cosmetic composition, and is a mixture of, as the lipophilic non-ionic emulsifier, a diester of a saturated carboxylic acid having 16 to 20 carbon atoms (fatty acid) with polyglycerol having 3 to 6 condensed glycerol moieties in its molecule, and, as a hydrophilic non-ionic co-emulsifier, a monoester of a saturated carboxylic acid having 16 to 20 carbon atoms (fatty acid) with polyethylene glycol (PEG) having 4 to 10 ethylene oxide (EO) moieties in its molecule, wherein the mass ratio of the lipophilic non-ionic emulsifier to the hydrophilic non-ionic co-emulsifier (lipophilic non-ionic emulsifier : hydrophilic non-ionic co-emulsifier) in the emulsifier component (iii) is 1 : ≥ 5; and
wherein the viscosity of the cosmetic composition is < 180 mPa·s.

(65) The cosmetic composition of (64), which has a viscosity of < 160 mPa·s.

(66) An applicator for cosmetic purpose impregnated and/or coated with a cosmetic composition as defined in any one of (48) to (65).

(67) The applicator of (66), which is a wipe or pad.

(68) The applicator of (66), which is a pad having the following properties in combination:

- It is a non-woven constituted of at least 95 % by mass of cotton fibres, expressed in terms of the mass of the pad;
- It has a thickness of between 1.9 and 2.1 mm;
- It has a grammage of between 170 and 190 g/$m^2$; and
- It has a decohesion force of at least 2.5 N.

## Claims

1. A use of a cosmetic composition for removing make-up, wherein the cosmetic composition is in the form of a water-in-oil emulsion and comprises:

   (i) an oil component having a polarity of at least 20 mN/m, and comprising a non-polar oil having a polarity greater than 30 mN/m and a low polarity oil having a polarity in the range of from 20 to 30 mN/m with a mass ratio of nonpolar oil to low polarity oil being in the range of from 1 to 5,
   (ii) water, and
   (iii) an emulsifier component comprising a lipophilic non-ionic emulsifier; wherein the viscosity of the cosmetic composition is ≤ 300 mPa·s preferably < 180 mPa·s;
   and

   wherein the cosmetic composition is coated onto and/or impregnated into an applicator for cosmetic purpose, wherein the content of the oil component (i) is 22 to 40 % by mass, relative to the total mass of the cosmetic composition.

2. The use according to Claim 1, wherein the applicator is a non-woven substrate.

3. The use according to Claim 2, wherein the non-woven substrate is a wipe or a pad.

4. The use according to any one of Claims 1 to 3, wherein the cosmetic composition has at least one of the following features (a) to (b):

   (a) The content of water (ii) therein is least 40 % by mass, relative to the total mass of the cosmetic composition;
   (b) The content of the emulsifier component (iii) therein is 0.1 to 10 % by mass, relative to the total mass of the cosmetic composition.

5. The use according to any one of Claims 1-4, wherein the non-polar oil is a $C_8$-$C_{32}$ hydrocarbon.

6. The use according to any one of Claims 1-5, wherein the low polarity oil is selected from the group consisting of a silicone oil and a dialkylcarbonate.

7. The use according to any one of Claims 1 to 6, wherein the oil component (i) is a mixture of $C_{12}$-$C_{16}$ alkane and silicone oil, in particular a mixture of $C_{12}$-$C_{16}$ alkane and a cyclic silicone.

8. The use according to any one of Claims 1 to 7, wherein the emulsifier component (iii) further comprises a hydrophilic emulsifier, which preferably is a non-ionic emulsifier.

9. The use according to any one of Claims 1 to 8, wherein the emulsifier(s) of the emulsifier component (iii) is/are selected from the group consisting of fatty acid esters of polyoxyalkylenes and fatty acid esters of polyglycerols.

10. A method for removing make-up comprising applying a cosmetic composition to the make-up, wherein the cosmetic composition is in the form of a water-in-oil emulsion and comprises:

    (i) an oil component having a polarity of at least 20 mN/m, and comprising a non-polar oil having a polarity greater than 30 mN/m and a low polarity oil having a polarity in the range of from 20 to 30 mN/m with a mass ratio of nonpolar oil to low polarity oil being in the range of from 1 to 5,
    (ii) water, and
    (iii) an emulsifier component comprising a lipophilic non-ionic emulsifier; and

    wherein the cosmetic composition is coated onto and/or impregnated into an applicator for cosmetic purpose, wherein the content of the oil component (i) is 22 to 40 % by mass, relative to the total mass of the cosmetic composition.

11. A cosmetic composition, being a make-up removing composition, which is in the form of a water-in-oil emulsion and comprises:

an oil component which is a mixture of $C_{12}$-$C_{16}$ alkane and a cyclic silicone, in particular $[(CH_3)_2SiO]_5$ and/or $[(CH_3)_2SiO]_6$, with a mass ratio of $C_{12}$-$C_{16}$ alkane to cyclic silicone being in the range of from 1 to 5,water, and an emulsifier component comprising a lipophilic non-ionic emulsifier,
wherein the content of the oil component (i) is 22 to 40 % by mass, relative to the total mass of the cosmetic composition.

**12.** The cosmetic composition of Claim 11, which has at least one of the following features (a) to (b):

(a) The content of water therein is least 40 % by mass, relative to the total mass of the cosmetic composition;
(b) The content of the emulsifier component therein is 0.1 to 10 % by mass, relative to the total mass of the cosmetic composition.

**13.** An applicator for cosmetic purpose impregnated and/or coated with a cosmetic composition as defined in Claim 11 or 12.

## Patentansprüche

**1.** Verwendung einer kosmetischen Zusammensetzung zum Entfernen von Make-up, wobei die kosmetische Zusammensetzung in der Form einer Wasser-in-Öl-Emulsion vorliegt und umfasst:

(i) eine Ölkomponente mit einer Polarität von wenigstens 20 mN/m, die ein nichtpolares Öl mit einer Polarität von höher als 30 mN/m und ein Öl mit einer niedrigen Polarität in dem Bereich von 20 bis 30 mN/m in einem Masseverhältnis von nichtpolarem Öl zu Öl mit niedriger Polarität in dem Bereich von 1 bis 5 umfasst,
(ii) Wasser und
(iii) eine Emulgatorkoponente, die einen lipophilen nichtionischen Emulgator umfasst;

wobei die Viskosität der kosmetischen Zusammensetzung ≤ 300 mPa·s beträgt, vorzugsweise < 180 mPa·s; und wobei die kosmetische Zusammensetzung auf einen Applikator für kosmetische Zwecke geschichtet und/oder darin imprägniert ist,
wobei der Gehalt an der Ölkomponente (i) 22 bis 40 Masse-% bezogen auf die Gesamtmasse der kosmetischen Zusammensetzung beträgt.

**2.** Verwendung gemäß Anspruch 1, wobei der Applikator ein Vliesstoffsubstrat ist.

**3.** Verwendung gemäß Anspruch 2, wobei das Vliesstoffsubstrat ein Wischtuch oder ein Kissen ist.

**4.** Verwendung gemäß einem der Ansprüche 1 bis 3, wobei die kosmetische Zusammensetzung wenigstens eines der folgenden Merkmale (a) bis (b) aufweist:

(a) der Gehalt an Wasser (ii) darin beträgt wenigstens 40 Masse-% bezogen auf die Gesamtmasse der kosmetischen Zusammensetzung;
(b) der Gehalt der Emulgatorkomponente (iii) darin beträgt 0,1 bis 10 Masse-% bezogen auf die Gesamtmasse der kosmetischen Zusammensetzung.

**5.** Verwendung gemäß einem der Ansprüche 1-4, wobei das nichtpolare Öl ein $C_8$-$C_{32}$-Kohlenwasserstoff ist.

**6.** Verwendung gemäß einem der Ansprüche 1-5, wobei das Öl mit niedriger Polarität ausgewählt ist aus der Gruppe bestehend aus einem Siliconöl und einem Dialkylcarbonat.

**7.** Verwendung gemäß einem der Ansprüche 1 bis 6, wobei die Ölkomponente (i) ein Gemisch von $C_{12}$-$C_{16}$-Alkan und Siliconöl ist, insbesondere ein Gemisch von $C_{12}$-$C_{16}$-Alkan und einem cyclischen Silicon.

**8.** Verwendung gemäß einem der Ansprüche 1 bis 7, wobei die Emulgatorkomponente (iii) ferner einen hydrophilen Emulgator umfasst, der vorzugsweise ein nichtionischer Emulgator ist.

**9.** Verwendung gemäß einem der Ansprüche 1 bis 8, wobei der/die Emulgator(en) der Emulgatorkomponente (iii)

ausgewählt ist/sind aus der Gruppe bestehend aus Fettsäureestern von Polyoxyalkylenen und Fettsäureestern von Polyglycerolen.

**10.** Verfahren zum Entfernen von Make-up, umfassend Aufbringen einer kosmetischen Zusammensetzung auf das Make-up,
wobei die kosmetische Zusammensetzung in der Form einer Wasser-in-Öl-Emulsion vorliegt und umfasst:

(i) eine Ölkomponente mit einer Polarität von wenigstens 20 mN/m, die ein nichtpolares Öl mit einer Polarität von höher als 30 mN/m und ein Öl mit einer niedrigen Polarität in dem Bereich von 20 bis 30 mN/m in einem Masseverhältnis von nichtpolarem Öl zu Öl mit niedriger Polarität in dem Bereich von 1 bis 5 umfasst,
(ii) Wasser und
(iii) eine Emulgatorkoponente, die einen lipophilen nichtionischen Emulgator umfasst; und

wobei die kosmetische Zusammensetzung auf einen Applikator für kosmetische Zwecke geschichtet und/oder darin imprägniert ist,
wobei der Gehalt an der Ölkomponente (i) 22 bis 40 Masse-% bezogen auf die Gesamtmasse der kosmetischen Zusammensetzung beträgt.

**11.** Kosmetische Zusammensetzung, die eine Make-up-Entfernungszusammensetzung ist, die in der Form einer Wasser-in-Öl-Emulsion vorliegt und umfasst:

eine Ölkomponente, die ein Gemisch von $C_{12}$-$C_{16}$-Alkan und einem cyclischen Silicon ist, insbesondere $[(CH_3)_2SiO]_5$ und/oder $[(CH_3)_2SiO]_6$, mit einem Masseverhältnis von $C_{12}$-$C_{16}$-Alkan zu cyclischem Silicon in dem Bereich von 1 bis 5, Wasser und
eine Emulgatorkoponente, die einen lipophilen nichtionischen Emulgator umfasst,
wobei der Gehalt an der Ölkomponente (i) 22 bis 40 Masse-% bezogen auf die Gesamtmasse der kosmetischen Zusammensetzung beträgt.

**12.** Kosmetische Zusammensetzung gemäß Anspruch 11, die wenigstens eines der folgenden Merkmale (a) bis (b) aufweist:

(a) der Gehalt an Wasser darin beträgt wenigstens 40 Masse-% bezogen auf die Gesamtmasse der kosmetischen Zusammensetzung;
(b) der Gehalt der Emulgatorkomponente darin beträgt 0,1 bis 10 Masse-% bezogen auf die Gesamtmasse der kosmetischen Zusammensetzung.

**13.** Applikator für kosmetische Zwecke, imprägniert und/oder beschichtet mit einer kosmetischen Zusammensetzung gemäß Anspruch 11 oder 12.

**Revendications**

**1.** Utilisation d'une composition cosmétique pour l'élimination de maquillage,
la composition cosmétique se trouvant sous forme d'une émulsion eau-dans-huile et comprenant :

(i) un composant huileux ayant une polarité d'au moins 20 mN/m et comprenant une huile non polaire ayant une polarité supérieure à 30 mN/m et une huile à polarité faible ayant une polarité dans la plage de 20 jusqu'à 30 mN/m, présentant un rapport de masse d'huile non polaire à huile à polarité faible se situant dans la plage de 1 jusqu'à 5,
(ii) de l'eau et
(iii) un composant émulsifiant comprenant un émulsifiant non ionique lipophile ; la viscosité de la composition cosmétique étant ≤ 300 mPa.s, préférablement étant < 180 mPa.s ;

et la composition cosmétique étant revêtue sur et/ou étant imprégnée dans un applicateur pour une utilisation cosmétique,
la teneur en composant huileux (i) étant de 22 jusqu'à 40% en masse, par rapport à la masse totale de la composition cosmétique.

**2.** Utilisation selon la revendication 1, l'applicateur étant un substrat non tissé.

**3.** Utilisation selon la revendication 2, le substrat non tissé étant une lingette ou un tampon.

**4.** Utilisation selon l'une quelconque des revendications 1 à 3, la composition cosmétique ayant au moins une des caractéristiques suivantes (a) jusqu'à (b) :

(a) sa teneur en eau (ii) est d'au moins 40% en masse, par rapport à la masse totale de la composition cosmétique ;
(b) sa teneur en composant émulsifiant (iii) est de 0,1 jusqu'à 10% en masse, par rapport à la masse totale de la composition cosmétique.

**5.** Utilisation selon l'une quelconque des revendications 1-4, l'huile non polaire étant un hydrocarbure en $C_{8-32}$.

**6.** Utilisation selon l'une quelconque des revendications 1-5, l'huile à polarité faible étant choisie dans le groupe constitué par une huile de silicone et un carbonate de dialkyle.

**7.** Utilisation selon l'une quelconque des revendications 1 à 6, le composant huileux (i) étant un mélange d'alcane en $C_{12-16}$ et d'huile de silicone, en particulier un mélange d'alcane en $C_{12-16}$ et d'une silicone cyclique.

**8.** Utilisation selon l'une quelconque des revendications 1 à 7, le composant émulsifiant (iii) comprenant en outre un émulsifiant hydrophile, qui est préférablement un émulsifiant non ionique.

**9.** Utilisation selon l'une quelconque des revendications 1 à 8, l'émulsifiant ou les émulsifiants du composant émulsifiant (iii) étant choisi(s) dans le groupe constitué par les esters d'acide gras de polyoxyalkylènes et les esters d'acide gras de polyglycérols.

**10.** Procédé pour l'élimination de maquillage comprenant l'application d'une composition cosmétique sur le maquillage, la composition cosmétique se trouvant sous forme d'une émulsion eau-dans-huile et comprenant :

(i) un composant huileux ayant une polarité d'au moins 20 mN/m et comprenant une huile non polaire ayant une polarité supérieure à 30 nM/m et comprenant une huile à polarité faible ayant une polarité dans la plage de 20 jusqu'à 30 mN/m, présentant un rapport de masse d'huile non polaire à huile à polarité faible se situant dans la plage de 1 jusqu'à 5,
(ii) de l'eau et
(iii) un composant émulsifiant comprenant un émulsifiant non ionique lipophile ; et

la composition cosmétique étant revêtue sur et/ou étant imprégnée dans un applicateur pour une utilisation cosmétique,
la teneur en composant huileux (i) étant de 22 jusqu'à 40% en masse, par rapport à la masse totale de la composition cosmétique.

**11.** Composition cosmétique, qui est une composition d'élimination de maquillage, se trouvant sous forme d'une émulsion eau-dans-huile et comprenant :

un composant huileux qui est un mélange d'un alcane en $C_{12-16}$ et d'une silicone cyclique, en particulier $[(CH_3)_2SiO]_5$ et/ou $[(CH_3)_2SiO]_6$, présentant un rapport de masse d'alcane en $C_{12-16}$ à silicone cyclique se situant dans la plage de 1 jusqu'à 5, de l'eau et
un composant émulsifiant comprenant un émulsifiant non ionique lipophile,
la teneur en composant huileux (i) étant de 22 jusqu'à 40% en masse, par rapport à la masse totale de la composition cosmétique.

**12.** Composition cosmétique selon la revendication 11, ayant au moins l'une des caractéristiques (a) jusqu'à (b) :

(a) sa teneur en eau (ii) est d'au moins 40% en masse, par rapport à la masse totale de la composition cosmétique ;
(b) sa teneur en composant émulsifiant est de 0,1 jusqu'à 10% en masse, par rapport à la masse totale de la composition cosmétique.

13. Applicateur pour utilisation cosmétique imprégné et/ou revêtu par une composition cosmétique telle que définie selon la revendication 11 ou 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1618925 A1
- US 6342469 B1
- US 6344204 B1
- WO 0156534 A1
- EP 1602359 A1
- GB 2000969 A
- US 20030027738 A1
- US 20050002994 A1
- EP 1555017 A1
- EP 1147760 A2
- EP 1340491 A2
- US 2004028633 A1
- EP 2505180 A1
- DE 10154627 A1
- US 6426079 B1
- EP 2198837 A1
- EP 2111840 A1
- EP 1621230 A1
- US 4011389 A
- US 3598865 A
- US 3721633 A
- US 3772269 A
- US 3640998 A
- US 3839318 A
- US 4223129 A
- WO 2006084991 A1
- WO 9417235 A
- US 5849647 A
- US 20100297191 A1


### Non-patent literature cited in the description


- *CHEMICAL ABSTRACTS,* 042-47-5, 4390-04-9, 93685-81-5, 13475-82-6, 68037-01-4
- *Chem. Rev.,* 1996, vol. 96, 951
- *CHEMICAL ABSTRACTS,* 1680-31-5
- *CHEMICAL ABSTRACTS,* 142-91-6, 110-27-0, 1680-31-5, 73398-61-5, 68171-38-0, 68171-33-5, 41669-30-1, 68411-27-8, 1190099-88-7, 15872-89-6, 1190099-88-7, 15872-89-6, 95912-86-0, 6938-94-9, 7491-02-3, 97338-28-8, 26942-95-0, 7360-38-5, 56519-71-2, 68956-68-3, 59130-69-7
- *Journal of the Society of Cosmetic Chemists,* 1954, vol. 4, 249-56
- Kirk-Othmer, Encyclopedia of Chemical Technology. Wiley, 1979, vol. 22, 333-432